# EUROPEAN PATENT APPLICATION

(11) **EP 4 830 827 A2**
(43) Date of publication of application: **28.10.2026**
(21) Application number: 26177661.1
(22) Date of filing: 01.07.2022
(51) Int. Cl.: B01J 19/00, B01L 3/00

(54) **REACTOR SYSTEM AND METHODS FOR USING THEREOF**

(30) Priority: 01.07.2021 US 202163217680 P; 18.08.2021 BE 202105657; 15.04.2022 US 202263331568 P; 30.06.2022 US 202217854536
(62) Document number(s) of the earlier application(s) in accordance with Art. 76 EPC: 22733683.1 / 4 363 551
(71) Applicant: Phoenix Biosciences SA, 1400 Nivelles (BE)
(72) Inventor: CASTILLO, José, 1400 Nivelles (BE); VANDEKERCKHOVE, Kristof, 1400 Nivelles (BE); RODRIGUEZ, Sébastien, 1400 Nivelles (BE); MAIRESSE, Bastien, 1400 Nivelles (BE)
(74) Representative: Brantsandpatents bv

(57) **Abstract**

Disclosed herein are systems, methods and devices for the continuous production and processing of compounds, including biopharmaceutical compounds. The system and devices are operated in an automated manner and capable of operation under Good Manufacturing Practice (GMP)-compliant conditions.

## Description

### BACKGROUND

Reactors refer to any manufactured device or system in which chemical or biochemical processes can be carried out, including the manufacturing of therapeutic, such as biological or small molecule, products. There is a need for reactors that enable continuous manufacturing process for Active Pharmaceutical Ingredients (API) and/or biopharmaceuticals through increased flexibility and lower operational costs.

### SUMMARY

The present disclosure provides methods and systems for the continuous production and processing of compounds, including biopharmaceutical compounds. Biopharmaceutical compounds may include therapeutic and/or prophylactic substances that require sequential processing steps, including synthesis, isolation, purification and packaging. In some aspects, the biopharmaceutical compounds include nucleic acid and biological macromolecules.

In an aspect, the present disclosure provides a system for producing a compound, comprising: a plurality of chambers, wherein each chamber of the plurality of chambers is configured to contain at least one reagent for producing the compound; at least one robotic arm configured to load each chamber with the at least one reagent; and at least one computer processor operatively coupled to the plurality of chambers and the at least one robotic arm, wherein the at least one robotic arm is programmable to: (a) fill a first chamber with the at least one reagent; (b) fill a second chamber after a time interval X with the at least one reagent; and (c) repeat step (b) until at least a portion of the plurality of chambers is filled with the at least one reagent, wherein the compound is produced and removed after a time interval Y from each of the portion of plurality of chambers in step (c), and wherein the system is programmable in at least a portion of the plurality of chambers to re-load, produce and remove the compound as in steps (a)-(c), thereby for a time period of at least 2Y continuously producing the compound within at least a portion of the plurality of chambers.

In some embodiments, the plurality of chambers is housed in a cartridge. In some embodiments, the plurality of chambers is disposed on a conveyor. In some embodiments, a number of the plurality of chambers is at least the time interval Y divided by the time interval X. In some embodiments, the compound is continuously produced for a time period of at least 3Y within at least a portion of the plurality of chambers. In some embodiments, the compound is continuously produced for a time period of at least 4Y within at least a portion of the plurality of chambers. In some embodiments, the compound is continuously produced for a time period of at least 5Y within at least a portion of the plurality of chambers. In some embodiments, the compound is continuously produced for a time period of at least 6Y within at least a portion of the plurality of chambers. In some embodiments, the compound is continuously produced for a time period of at least 7Y within at least a portion of the plurality of chambers. In some embodiments, the compound is continuously produced for a time period of at least 8Y within at least a portion of the plurality of chambers. In some embodiments, the compound is continuously produced for a time period of at least 9Y within at least a portion of the plurality of chambers. In some embodiments, the compound is continuously produced for a time period of at least 10Y within at least a portion of the plurality of chambers. In some embodiments, the compound is continuously produced for a time period of at least 20Y within at least a portion of the plurality of chambers. In some embodiments, the compound is continuously produced for a time period of at least 30Y within at least a portion of the plurality of chambers. In some embodiments, the compound is continuously produced for a time period of at least 40Y within at least a portion of the plurality of chambers. In some embodiments, the compound is continuously produced for a time period of at least 50Y within at least a portion of the plurality of chambers. In some embodiments, the compound is continuously produced for a time period of at least 60Y within at least a portion of the plurality of chambers. In some embodiments, the compound is continuously produced for a time period of at least 70Y within at least a portion of the plurality of chambers. In some embodiments, the compound is continuously produced for a time period of at least 80Y within at least a portion of the plurality of chambers. In some embodiments, the compound is continuously produced for a time period of at least 90Y within at least a portion of the plurality of chambers. In some embodiments, the compound is continuously produced for a time period of at least 100Y within at least a portion of the plurality of chambers. In some embodiments, the compound is continuously produced for a time period of at least 200Y within at least a portion of the plurality of chambers. In some embodiments, the compound is continuously produced for a time period of at least 300Y within at least a portion of the plurality of chambers. In some embodiments, the compound is continuously produced for a time period of at least 400Y within at least a portion of the plurality of chambers. In some embodiments, the compound is continuously produced for a time period of at least 500Y within at least a portion of the plurality of chambers. In some embodiments, the compound is continuously produced for a time period of at least more than 5Y within at least a portion of the plurality of chambers. In some embodiments, the compound is continuously produced for a time period of at least more than 6Y within at least a portion of the plurality of chambers. In some embodiments, the compound is continuously produced for a time period of at least more than 7Y within at least a portion of the plurality of chambers. In some embodiments, the compound is continuously produced for a time period of at least more than 8Y within at least a portion of the plurality of chambers. In some embodiments, the compound is continuously produced for a time period of at least more than 9Y within at least a portion of the plurality of chambers. In some embodiments, the compound is continuously produced for a time period of at least more than 10Y within at least a portion of the plurality of chambers. In some embodiments, the compound is continuously produced for a time period of at least more than 20Y within at least a portion of the plurality of chambers. In some embodiments, the compound is continuously produced for a time period of at least more than 30Y within at least a portion of the plurality of chambers. In some embodiments, the compound is continuously produced for a time period of at least more than 40Y within at least a portion of the plurality of chambers. In some embodiments, the compound is continuously produced for a time period of at least more than 50Y within at least a portion of the plurality of chambers. In some embodiments, the compound is continuously produced for a time period of at least more than 60Y within at least a portion of the plurality of chambers. In some embodiments, the compound is continuously produced for a time period of at least more than 70Y within at least a portion of the plurality of chambers. In some embodiments, the compound is continuously produced for a time period of at least more than 80Y within at least a portion of the plurality of chambers. In some embodiments, the compound is continuously produced for a time period of at least more than 90Y within at least a portion of the plurality of chambers. In some embodiments, the compound is continuously produced for a time period of at least more than 100Y within at least a portion of the plurality of chambers. In some embodiments, the compound is continuously produced for a time period of at least more than 200Y within at least a portion of the plurality of chambers. In some embodiments, the compound is continuously produced for a time period of at least more than 300Y within at least a portion of the plurality of chambers. In some embodiments, the compound is continuously produced for a time period of at least more than 400Y within at least a portion of the plurality of chambers. In some embodiments, the compound is continuously produced for a time period of at least more than 500Y within at least a portion of the plurality of chambers. In some embodiments, the cartridge comprises at least about 2 chambers. In some embodiments, the cartridge comprises at least about 3 chambers. In some embodiments, the cartridge comprises at least about 4 chambers. In some embodiments, the cartridge comprises at least about 5 chambers. In some embodiments, the cartridge comprises at least about 6 chambers. In some embodiments, the cartridge comprises at least about 7 chambers. In some embodiments, the cartridge comprises at least about 8 chambers. In some embodiments, the cartridge comprises at least about 9 chambers. In some embodiments, the cartridge comprises at least about 10 chambers. In some embodiments, the cartridge comprises at least about 12 chambers. In some embodiments, the cartridge comprises at least about 20 chambers. In some embodiments, the cartridge comprises at least about 24 chambers. In some embodiments, the cartridge comprises at least about 30 chambers. In some embodiments, the cartridge comprises at least about 36 chambers. In some embodiments, the cartridge comprises at least about 40 chambers. In some embodiments, the cartridge comprises at least about 48 chambers. In some embodiments, the cartridge comprises at least about 50 chambers. In some embodiments, the chamber is removable from the cartridge. In some embodiments, the chamber is not removable from the cartridge. In some embodiments, the chamber is removable from the conveyor. In some embodiments, the chamber is not removable from the conveyor. In some embodiments, the chamber comprises a volume of about at least 0.1 ml to at least about 1000 mL. In some embodiments, the chamber comprises a volume of about at least 1 mL. In some embodiments, the chamber comprises a volume of about at least 5 mL. In some embodiments, the chamber comprises a volume of about at least 10 mL. In some embodiments, the chamber comprises a volume of at least about 10 mL. In some embodiments, the chamber is pie wedge shaped. In some embodiments, the chamber is regular or irregular polygon shaped. In some embodiments, the chamber is regular or irregular hexagon shaped. In some embodiments, the chamber is circular shaped. In some embodiments, the chamber is regular or elliptic shaped. In some embodiments, the chamber comprises at least one baffle. In some embodiments, the first chamber touches at least one side wall of the second chamber. In some embodiments, the first chamber does not touch any side wall of the second chamber. In some embodiments, when the last chamber of the plurality of chambers is filled with the at least one reagent, the compound is produced and removed from the first chamber of the plurality of chambers.

In some embodiments, the time interval Y is at least the time interval X times the number of the plurality of chambers. In some embodiments, the time interval X comprises at least about 2 minutes. In some embodiments, the time interval X comprises at least about 3 minutes. In some embodiments, the time interval X comprises at least about 4 minutes. In some embodiments, the time interval X comprises at least about 5 minutes. In some embodiments, the time interval X comprises at least about 10 minutes. In some embodiments, the time interval X comprises at least about 15 minutes. In some embodiments, the time interval X comprises at least about 20 minutes. In some embodiments, the time interval X comprises at least about 25 minutes. In some embodiments, the time interval X comprises at least about 30 minutes. In some embodiments, the time interval X comprises at least about 35 minutes. In some embodiments, the time interval X comprises at least about 40 minutes. In some embodiments, the time interval X comprises at least about 45 minutes. In some embodiments, the time interval X comprises at least about 50 minutes. In some embodiments, the time interval X comprises at least about 55 minutes. In some embodiments, the time interval X comprises at least about 60 minutes. In some embodiments, the time interval Y comprises at least about 30 minutes. In some embodiments, the time interval Y comprises at least about 60 minutes. In some embodiments, the time interval Y comprises at least about 75 minutes. In some embodiments the time interval Y comprises at least about 90 minutes. In some embodiments, the time interval Y comprises at least about 120 minutes. In some embodiments, the time interval Y comprises at least about 180 minutes. In some embodiments, the time interval Y comprises at least about 210 minutes. In some embodiments, the time interval Y comprises at least about 240 minutes. In some embodiments, the time interval Y comprises at least about 270 minutes. In some embodiments, the time interval Y comprises at least about 300 minutes. In some embodiments, the time interval Y comprises at least about 330 minutes. In some embodiments, the time interval Y comprises at least about 360 minutes. In some embodiments, the time interval Y comprises at least about 420 minutes. In some embodiments, the time interval Y comprises at least about 480 minutes. In some embodiments, the time interval Y comprises at least about 720 minutes. In some embodiments, the time interval Y comprises at least about 1,440 minutes. In some embodiments, the compound is removed by the at least one robotic arm. In some embodiments, the compound is removed by a pressurized system. In some embodiments, the pressurized system comprises a negative pressure. In some embodiments, the pressurized system comprises a pump configured to remove a medium comprising the compound. In some embodiments, the pump is in fluid communication with a chamber. In some embodiments, the first chamber is not in fluid communication with the second chamber. In some embodiments, the chamber is configured to conduct an in vitro transcription (IVT) reaction. In some embodiments, the compound is a RNA molecule. In some embodiments, a reagent comprises a DNA template. In some embodiments, the DNA template is circular. In some embodiments, the DNA template is linear. In some embodiments, the chamber is configured to conduct an in vitro translation. In some embodiments, the in vitro translation is cell dependent. In some embodiments, the in vitro translation is cell free. In some embodiments, the chamber is not designed to culture a living cell.

In some embodiments, the chamber is configured to monitor and/or regulate a pH value of a medium comprising the at least one reagent. In some embodiments, the chamber is configured to monitor and /or regulate a temperature of a medium comprising the at least one reagent or an atmosphere within the chamber. In some embodiments, the chamber is configured to monitor and/or regulate a salt concentration of a medium comprising the at least one reagent. In some embodiments, the chamber is configured to monitor and/or regulate osmolarity of a medium comprising the at least one reagent. In some embodiments, the chamber is configured to monitor and/or regulate conductivity of a medium comprising the at least one reagent. In some embodiments, the chamber is configured to monitor and/or regulate a turbidity of the medium comprising the at least one reagent. In some embodiments, the chamber is configured to monitor and /or regulate a volume of the medium comprising the at least one reagent. In some embodiments, the chamber is configured to monitor and/or regulate a humidity of the atmosphere within the chamber. In some embodiments, the chamber is configured to monitor and/or regulate an O₂ concentration or CO₂ concentration of the atmosphere within the chamber. In some embodiments, the chamber is configured to monitor and/or regulate a concentration of a molecule comprised in the medium utilizing a spectroscopic device. In some embodiments, the spectroscopic device comprises an Infrared spectrometer, a Raman spectrometer, or a UV spectrometer.

In some embodiments, the system is programmable to inspect the medium. In some embodiments, the chamber comprises an agitation device. In some embodiments, the agitation device comprises a baffle, a magnetic bar, an impeller, or a bead. In some embodiments, the cartridge is programmable to shake to mix the medium. In some embodiments, the chamber comprises a lid. In some embodiments, the lid is removable. In some embodiments, the lid is not removable. In some embodiments, the lid comprises at least one opening. In some embodiments, a chamber is configured to detect a contamination of the at least one reagent. In some embodiments, the system further comprises at least one intermediate storage vessel. In some embodiments, the intermediate storage vessel is configured to store the compound. In some embodiments, the system comprises a purification system. In some embodiments, the purification system comprises an affinity purification system. In some embodiments, the purification system comprises an ion exchange system. In some embodiments, the purification system comprises a selective precipitation system. In some embodiments, the purification system comprises a chromatography system. In some embodiments, the purification system comprises a tangential flow filtration device or a dead-end filtration device. In some embodiments, the intermediate storage vessel is not in fluid communication with the chamber. In some embodiments, the storage vessel is configured to detect a contamination of the compound.

In some embodiments, the system further comprises a modular unit. In some embodiments, the modular unit comprises a compartment. In some embodiments, the compartment comprises at least one cartridge. In some embodiments, the at least one cartridge is stackable. In some embodiments, the modular unit comprises at least one carousel. In some embodiments, the at least on carousel is stackable. In some embodiments, the modular unit comprises at least one conveyor. In some embodiments, the at least one conveyor is stackable. In some embodiments, the modular unit is movable. In some embodiments, the modular unit is stackable. In some embodiments, the modular unit comprises a plurality of receiving containers. In some embodiments, the receiving container is configured to contain at least one reagent. In some embodiments, the modular unit comprises a docking station configured to receive a receiving container, a cartridge, a carousel, or a conveyor. In some embodiments the modular unit is mobile. In some embodiments, the modular unit is non-mobile. In some embodiments, the modular unit is configured to agitate contents (e.g., medium comprise in a chamber) disposed therein. In some cases, the modular unit is configured to shake or oscillate, thereby mixing contents disposed therein. In some embodiments, the modular unit is configured to regulate one or more properties of a chamber, cartridge, conveyor, or carousel disposed therein. In some embodiments, the modular unit is configured to regulate a temperature of the cartridge or chamber. In some embodiments, the system further comprises a second robotic arm. In some embodiments, the system further comprises a third robotic arm. In some embodiments, the system further comprises a waste collecting vessel. In some embodiments, the system further comprises a HVAC system comprising a series of HEPA filters to protect the compound within the chamber. In some embodiments, a chamber, a storage vessel, and a robotic arm are configured respectively to transmit data.

In another aspect, the present disclosure provides a method for producing a compound, comprising: (a) providing a plurality of chambers, wherein a chamber of the plurality of chambers comprises a medium comprising at least one reagent; and (b) using at least one computer processor to generate instructions to: (i) fill a chamber with the medium; (ii) repeat (i) until at least a portion of the plurality of chambers is filled with the medium; (iii) synthesize the compound in a chamber that is filled with the medium and remove the compound from the chamber that is filled with the medium after a time interval Y; and (iv) continuously re-load, synthesize, and remove the compound. In some embodiments, the method further comprises rinsing or washing the chamber subsequent to (iii). In some embodiments, the method further comprises providing a robotic arm coupled to the at least one computer processor and configured to receive the instructions in (b). In some embodiments, the method further comprises filling a subsequent chamber after a time interval X. In some embodiments, the time interval Y is at least the time interval X times the number of the plurality of chambers. In some embodiments, the method further comprises the at least one computer processor to generate instructions to (1) fill a chamber with the medium and remove the medium comprising the compound and (2) repeat (1) in the same chamber until receiving an input for stop.

In some embodiments, the method further comprises filling a first chamber with a first medium comprising a first reagent and a second chamber with a second medium comprising a second reagent. In some embodiments, the first reagent is different from the second reagent. In some embodiments, the first reagent is the same as the second reagent. In some embodiments, the first chamber and the second chamber comprise a substantially same temperature. In some embodiments, the first chamber and the second chamber comprise different temperatures. In some embodiments, the first chamber and the second chamber comprise a substantially same reaction time. In some embodiments, the first chamber and the second chamber comprise different reaction times. In some embodiments, the method further comprises monitoring a temperature of the medium. In some embodiments, the method further comprises monitoring a temperature of an atmosphere within a chamber. In some embodiments, the method further comprises monitoring a concentration of a molecule comprised within the medium. In some embodiments, the method further comprises monitoring an oxygen concentration of the atmosphere within the chamber. In some embodiments, the method further comprises monitoring a carbon dioxide concentration of the atmosphere within the chamber. In some embodiments, the method further comprises monitoring concentration of gas dissolved in the medium within the chamber. In some embodiments, the dissolved gas comprises carbon dioxide. In some embodiments, the dissolved gas comprises oxygen. In some embodiments, the method further comprises monitoring a volume level of a medium. In some embodiments, the method further comprises monitoring a turbidity of a medium. In some embodiments, the method further comprises monitoring a pH value of a medium. In some embodiments, the method further comprises monitoring a humidity of the atmosphere within the chamber. In some embodiments, the method further comprises detecting a contamination in a chamber. In some embodiments, the method further comprises purifying the compound.

In another aspect, the present disclosure provides a method for producing a compound comprising: (i) providing a plurality of chambers, (ii) filling a first chamber with a medium, wherein the medium comprises at least one reagent; (iii) filling a further chamber after a time interval X with the medium; (iv) repeating step (iii) until at least a portion of the plurality of chambers is filled with the medium; and (v) producing and removing the compound after a time interval Y from each of the portion of the plurality of chambers in step (iv), thereby for a time period of at least 2Y continuously producing the compound within the at least a portion of the plurality of chambers.

In some embodiments, the method further comprises rinsing or washing the first chamber subsequent to (v). In some embodiments, said filling is performed by at least one robotic arm. In some embodiments, the time interval Y is at least the time interval X times the number of the plurality of chambers. In some embodiments, the method further comprises filling the first chamber with a first medium comprising a first reagent and thefurther chamber with a second medium comprising a second reagent. In some embodiments, the first reagent is different from the second reagent. In some embodiments, the first reagent is the same as the second reagent. In some embodiments, the first chamber and the further chamber comprise a substantially same temperature. In some embodiments, the first chamber and the further chamber comprise different temperatures. In some embodiments, the first chamber and the further chamber comprise a substantially same reaction time. In some embodiments, the first chamber and the further chamber comprise different reaction times. In some embodiments, the method further comprises monitoring a temperature of the medium. In some embodiments, the method further comprises monitoring a temperature of an atmosphere within the first chamber. In some embodiments, the method further comprises monitoring a concentration of a molecule comprised within the medium. In some embodiments, the method further comprises monitoring an oxygen concentration of the atmosphere within the first chamber. In some embodiments, the method further comprises monitoring a carbon dioxide concentration of the atmosphere within the first chamber. In some embodiments, the method further comprises monitoring a concentration of gas dissolved in the medium within the first chamber. In some embodiments, the method further comprises monitoring a volume level of the medium in the first chamber. In some embodiments, the method further comprises monitoring a turbidity of the medium. In some embodiments, the method further comprises monitoring a pH value of the medium. In some embodiments, the method further comprises monitoring a humidity of the atmosphere within the chamber. In some embodiments, the method further comprises detecting a contamination in a chamber of the plurality of chambers. In some embodiments, the method further comprises purifying the compound. In some embodiments, said compound is a nucleotide, such as RNA or DNA.

In another aspect, the present disclosure provides for a chamber for the production of a compound, the chamber comprising a bottom portion, a body portion comprising an inner volume configured to hold at least one reagent or a compound, an open end configured to receive at least one reagent, wherein at least part of said body portion comprises a flat polygonal surface configured to position and/or support a sensor in the reaction chamber. The chamber of claim 121, wherein the body portion comprises at least three flat polygonal surfaces.

In some embodiments, a cross-section of the body portion comprises a polygonal shape. In some embodiments, the polygonal shape comprises a trigonal, tetragonal, pentagonal or hexagonal shape. In some embodiments, the cross-section of the body portion is hexagonal. In some embodiments, the open end is configured to receive a removable lid for at least partially closing the open end. In some embodiments, the bottom is rounded. In some embodiments, the chamber comprises a chamber volume of about 0.1 mL to about 1000 mL. In some embodiments, the chamber is configured for single use or multiple uses. In some embodiments, the chamber further comprises a scannable identification (ID) mechanism. In some embodiments, the scannable ID mechanism comprises an RFID.

In another aspect, the present disclosure provides for an assembly comprising a lid configured to at least partially close the open end of a chamber.

In some embodiments, the lid comprises a removable lid. In some embodiments, the lid comprises a push-on lid. In some embodiments, the lid comprises a screw lid. In some embodiments, the lid is glued to at least a portion of the chamber. In some embodiments, the lid comprises an opening. In some embodiments, the opening is centrally located within the lid. In some embodiments, the lid comprises a puncturable membrane. In some embodiments, the puncturable membrane comprises an elastic membrane.

In another aspect, the present disclosure provides for a cartridge comprising a plurality of chambers. In some embodiments, the plurality of chambers comprises between 2 and 20 chambers. In some embodiments, the plurality of chambers comprises between 2 and 10 chambers. In some embodiments, the cartridge further comprises at least one aperture. In some embodiments, the at least one aperture is configured to engage with a gripping mechanism. In some embodiments, the cartridge is configured to engage with a robotic arm and/or a handling device. In some embodiments, the robotic arm and/or the handling device comprises the gripping mechanism. In some embodiments, the cartridge comprises at least two apertures, wherein the at least two apertures are configured to engage with the gripping mechanism. In some embodiments, the at least one aperture is positioned at an upper surface of the cartridge. In some embodiments, the cartridge further comprises a scannable ID mechanism. In some embodiments, the scannable ID mechanism comprises the RFID.

In another aspect, the present disclosure provides a method comprising: (a) providing or obtaining the chamber as described herein; and producing a nucleic acid in the chamber.

In some embodiments, the nucleic acid is a ribonucleic acid (RNA).In some embodiments, the chamber is comprised in an assembly as described herein. In some embodiments, the chamber is comprised in a cartridge as described herein.

In another aspect, the present disclosure provides a device for producing a nucleic acid compound, the device comprising: a plurality of chambers, wherein each chamber of the plurality of chambers is configured to contain at least one reagent for producing the nucleic acid compound; at least one robotic arm configured to load each chamber with the at least one reagent; and at least one computer processor operatively coupled to the plurality of chambers and the at least one robotic arm, wherein the at least one robotic arm is programmed to regularly fill the plurality of chambers with at least one reagent after a time period Y, wherein the device is programmed to remove nucleic acid compound produced from each of the plurality of chambers after a time interval Y, and wherein the device is further programmed to re-load, produce and remove the compound in a continuous manner thereby for a time period of at least 2Y.

In another aspect, the present disclosure provides a device for producing a nucleic acid compound, the device comprising: a plurality of chambers, wherein each chamber of the plurality of chambers is configured to contain at least one reagent for producing the nucleic acid compound and the plurality of chambers is contained within a compartment capable of moving said plurality of chambers within the device; at least one robotic arm configured to load each chamber with the at least one reagent; and at least one computer processor operatively coupled to the plurality of chambers and the at least one robotic arm, wherein the at least one robotic arm is programmed to regularly fill the plurality of chambers with at least one reagent after a time period Y, wherein the device is programmed to remove nucleic acid compound produced from each of the plurality of chambers after a time interval Y.

In some embodiments, the device is further programmed to wash each of the plurality of chambers after removing the nucleic acid compound produced from each of the plurality of chambers after the time interval Y. In some embodiments, the robotic arm is further programmed to (1) fill each chamber with a medium comprising the at least one reagent and remove the medium comprising the nucleic acid compound and (2) repeat (1) in the same chamber until receiving an input for stop. In some embodiments, the device further comprises a second plurality of chambers configured to purify the nucleic acid compound. In some embodiments, the at least one robotic arm is further programmed to transport the nucleic acid compound to a chamber of the second plurality of chambers. In some embodiments, the device further comprises a second robotic arm, wherein the second robotic arm is programmed to transport the nucleic acid compound to a chamber of the second plurality of chambers. In some embodiments, the second plurality of chambers is comprised in a second compartment capable of moving the second plurality of chambers within the device. In some embodiments, the compartment comprises a conveyor or a carousel. In some embodiments, the second compartment comprises a second conveyor or carousel. In some embodiments, a speed of the compartment is determined at least in part by the time period Y.

Described herein are various embodiments of a chamber for the production of a compound, the chamber comprising a bottom portion, a body portion comprising an inner volume configured to hold at least one reagent or a compound, an open end configured to receive at least one reagent, wherein at least part of said body portion comprises a flat polygonal surface configured to position and/or support a sensor in the reaction chamber. In some embodiments, the body portion comprises at least three flat polygonal surfaces. In some embodiments, a cross-section of the body portion is polygonal shape. In some embodiments, polygonal shape comprises a trigonal, tetragonal, pentagonal or hexagonal shape. In some embodiments, the cross-section of the body portion is hexagonal. In some embodiments, the open end is configured to receive a removable lid for at least partially closing the open end. In some embodiments, the bottom is rounded. In some embodiments, the chamber comprises a chamber volume of about 0.1 mL to about 1000 mL. In some embodiments, the chamber is configured for single use or multiple uses. The chamber of any one of the preceding claims, further comprising a scannable identification (ID) mechanism. In some embodiments, the scannable ID mechanism comprises an RFID.

Described herein are various embodiments of an assembly comprising a lid configured to at least partially close the open end of the chamber. In some embodiments, the lid comprises a removable lid. In some embodiments, the lid comprises a push-on lid. In some embodiments, the lid comprises a screw lid. In some embodiments, the lid is bonded (e.g., adhered) to another portion of the assembly. In some embodiments, the lid is bonded to another portion of the assembly with use of an adhesive. In some embodiments, the lid is glued to another portion of the assembly. In some embodiments, the lid comprises an opening. In some embodiments, the opening is centrally located within the lid. In some embodiments, the lid comprises a puncturable membrane. In some embodiments, the puncturable membrane comprises an elastic membrane.

Described herein are various embodiments of a cartridge comprising a plurality of chambers. In some embodiments, the at least one aperture comprises between 2 and 20 chambers. In some embodiments, the at least one aperture comprises between 2 and 10 chambers. In some embodiments, the cartridge further comprises at least one aperture. In some embodiments, the at least one aperture is configured to engage with a gripping mechanism. In some embodiments, the cartridge is configured to engage with a robotic arm and/or a handling device. In some embodiments, the robotic arm and/or the handling device comprises the gripping mechanism. In some embodiments, the cartridge comprises at least two apertures, wherein the at least two apertures are configured to engage with the gripping mechanism. In some embodiments, the at least one aperture is positioned at an upper surface of the cartridge. The cartridge of any one of the preceding claims, further comprising the scannable ID mechanism. In some embodiments, the scannable ID mechanism comprises the RFID.

Described herein are various embodiments of methods and devices comprising: providing or obtaining the chamber as described in any one of the preceding claims; and producing a nucleic acid. In some embodiments, the nucleic acid is a ribonucleic acid (RNA). In yet other embodiments, the methods and devices as disclosed herein comprise a hexagonally packed cartridge, the cartridge comprising: a plurality of hexagonal chambers, or tubes, the hexagonal chambers, or tubes configured for in vitro transcription (IVT) and down-stream processing (DSP), and wherein the cartridge is configured for each of the hexagonal chambers of the plurality to be arranged in a hexagonally-packed configuration. In some embodiments, the DSP comprises post-transcriptional capping and/or purification of the RNA molecule.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** depicts an embodiment of the cartridge, chambers and robotic arm of a system, wherein the chambers are arranged in a geometrical configuration, for example in a circular manner, divided from each other by upstanding walls.
**FIG. 2** depicts another embodiment of the cartridge, chambers and robotic arm of a system, wherein the chambers are arranged in a geometrical configuration, for example in a rectangular manner.
**FIG. 3** depicts another embodiment of the cartridge, chambers and robotic arm of a system, wherein the chambers are arranged in a geometrical configuration, for example in a honeycomb structure.
**FIG. 4** depicts a top view and movement of one embodiment of the cartridge and chambers of a system.
**FIG. 5** depicts another embodiment of cartridges, chambers and robotic arms of a system, which allows to work with single and/or several cartridges in parallel.
**FIG. 6** is a cross-sectional view of an embodiment depicting the stackability of multiple cartridges of a system in a vertical arrangement.
**FIG. 7** depicts another embodiment of a plurality of chambers and robotic arm of a system, wherein chambers are configured to move on a conveyor belt powered by one or more powered pulleys.
**FIG. 8** depicts the combination of a cartridge, intermediate vessel and down-stream processing components of a system.
**FIGS. 9A** and **9B** depicts a modular combination of components of a system in accordance with aspects of the present disclosure.
**FIG. 10** depicts a computer system that is programmed or otherwise configured to implement methods provided herein.
**FIG. 11** depicts a chamber in accordance with some embodiments of the present disclosure.
**FIGS. 12A-12J** depict various cartridges in accordance with some embodiments of the present disclosure.
**FIG. 13** depicts an overview of an experiment.
**FIG. 14** depicts an overview of an in vitro transcription (IVT) process carried out as part of an experiment described herein.
**FIGS. 15A-15B** show the results of quantitation of RNA synthesized in accordance with methods as disclosed herein.
**FIGS. 16A-16B** show the results of quantitation of residual nucleic acids and protein in a reactor as described herein.
**FIG. 17** shows the proportion of double stranded RNA (dsRNA) to total RNA in a sample processed as part of an experiment described herein.
**FIGS. 18A-18C** depict results from computer simulations of systems and methods described herein.

### DETAILED DESCRIPTION

There is need for the development of bioreactors which enable a continuous manufacturing process for pharmaceuticals, biochemical and biological compounds, including biopharmaceuticals. Continuous manufacturing has many advantages: The equipment is utilized more efficiently as all unit operations are active at the same time, compared with a batch process which requires significant idle time of equipment. Less human intervention is also contemplated, reducing operational cost and human error. Continuous manufacturing may also enable a smaller footprint, potentially reducing operational costs. Lastly, pilot-scale processes, which may also be used for clinical supply, can transition into commercial manufacturing by increasing the run time. This eliminates scale-up and its associated construction and validation steps and speeds up time to market.

Accordingly, disclosed herein are systems, methods and devices for the continuous production and processing of compounds, including biopharmaceutical compounds. The system and devices are operated in an automated manner and capable of operation under Good Manufacturing Practice (GMP)-compliant conditions.

Unless defined otherwise, all terms of art, notations and other technical and scientific terms or terminology used herein are intended to have the same meaning as is commonly understood by one of ordinary skill in the art to which the claimed subject matter pertains. In some embodiments, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used in the specification and claims, the singular forms "a", "an" and "the" include plural references unless the context clearly dictates otherwise. For example, the term "a sample" includes a plurality of samples, including mixtures thereof.

The terms "determining," "measuring," "evaluating," "assessing," "assaying," and "analyzing" are often used interchangeably herein to generally refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

The terms "subject," "individual," or "patient" are often used interchangeably herein. A "subject" can generally refer to a biological entity containing expressed genetic materials. The biological entity can be a plant, animal, or microorganism, including, for example, bacteria, viruses, fungi, and protozoa. The subject can be tissues, cells and their progeny of a biological entity obtained in vivo or cultured in vitro. The subject can be a mammal. The mammal can be a human. The subject may be diagnosed or suspected of being at high risk for a disease. In some embodiments, the subject is not necessarily diagnosed or suspected of being at high risk for the disease.

The term *"in vivo"* is generally used to describe an event that takes place in a subject's body.

The term *"ex vivo"* is generally used to describe an event that takes place outside of a subject's body. An *ex vivo* assay is not performed on a subject. Rather, it is performed upon a sample separate from a subject. An example of an *ex vivo* assay performed on a sample is an *"in vitro"* assay.

The term *"in vitro"* is used to describe an event that takes places contained in a container for holding laboratory reagent such that it is separated from the biological source from which the material is obtained. *In vitro* assays can encompass cell-based assays in which living or dead cells are employed. *In vitro* assays can also encompass a cell-free assay in which no intact cells are employed.

As used herein, the term "about" a number generally refers to that number plus or minus 10% of that number. The term "about" a range refers to that range minus 10% of its lowest value and plus 10% of its greatest value.

As used herein, the terms "therapy," or "therapeutic," or other derived terms are generally used in reference to a pharmaceutical or other intervention regimen for obtaining beneficial or desired results in the recipient. Beneficial or desired results include but are not limited to a therapeutic benefit and/or a prophylactic benefit. A therapeutic benefit may refer to eradication or amelioration of symptoms or of an underlying disorder being treated. Also, a therapeutic benefit can be achieved with the eradication or amelioration of one or more of the physiological symptoms associated with the underlying disorder such that an improvement is observed in the subject, notwithstanding that the subject may still be afflicted with the underlying disorder. A prophylactic effect may include delaying, preventing, or eliminating the appearance of a disease or condition, delaying or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

Described herein are various embodiments of reactor systems. In some embodiments, the systems may comprise a chamber; a cartridge; a device for purification and/or separation of a compound; a lid; a sensor; or a combination thereof. In some embodiments, the chamber may comprise a bottom portion, a body portion comprising an inner volume, the inner volume configured to hold at least one reagent or a compound. In some embodiments, the chamber comprises an open end configured to receive at least one reagent, wherein at least part of the body portion comprises a flat polygonal surface configured to allow positioning and/or supporting a sensor in the chamber. In some embodiments, at least one chamber is included within a cartridge.

In some embodiments, various methods may comprise producing and/or synthesizing compounds in accordance with some embodiments. In some embodiments, the compound may comprise a chemical compound or biochemical compounds. In some embodiments, various methods may comprise manufacturing compound comprising a therapeutic, such as a biological or small molecule compound. In some embodiments, the compound may be an RNA molecule, obtained by an in vitro transcription method and further processed downstream, for instance by means of isolation, purification, and/or packaging.

Described herein are various embodiments of a reactor system comprising: a chamber; and a cartridge. In some embodiments, the chamber is configured for production of a compound. In some embodiments, the chamber comprises: a bottom portion; a body portion, the body portion comprising an inner volume configured to hold at least one reagent or a compound; an open end configured to receive at least one reagent, wherein at least part of the body portion comprises a flat polygonal surface configured to position and/or support a sensor in the chamber. In some embodiments, the body portion comprises at least three flat polygonal surfaces. In some embodiments, a cross-section of the body portion comprises a polygonal shape. In some embodiments, the polygonal shape comprises a trigonal, tetragonal, pentagonal or hexagonal shape. In some embodiments, the cross-section of the body portion comprises a hexagonal shape. In some embodiments, the open end is configured to receive a removable lid for at least partially closing the open end. In some embodiments, the bottom is rounded. In some embodiments, the chamber comprises a chamber volume of 0.1 mL to 1000 mL. In some embodiments, the chamber may be configured for single use or multiple uses. In some embodiments, the system further comprises a scannable identification mechanism. In some embodiments, the scannable identification mechanism comprises an RFID. In some embodiments, the chamber comprises a lid configured to at least partially close the open end of the chamber. In some embodiments, the lid comprises a removable lid. In some embodiments, the lid comprises a push-on lid. In some embodiments, the lid comprises a screw lid. In some embodiments, the lid is bonded to at least part of the chamber. In some embodiments, the lid is adhered to at least a part of the chamber. In some embodiments, the lid comprises a glued lid. In some embodiments, the lid comprises an opening. In some embodiments, the lid comprises the opening, wherein the opening is centrally located within the lid. In some embodiments, the lid comprises a puncturable membrane. In some embodiments, the puncturable membrane comprises an elastic membrane. In some embodiments, the cartridge comprises a plurality of chambers. In some embodiments, the cartridge comprises 2 to 20 chambers. In some embodiments, the cartridge comprises 2 to 10 chambers. In some embodiments, the cartridge comprises at least one aperture. In some embodiments, the at least one aperture is configured to engage with a gripping mechanism. In some embodiments, the system further comprises a robotic arm and/or a handling device. In some embodiments, the robotic arm and/or the handling device comprises the gripping mechanism. In some embodiments, the cartridge comprises at least two apertures, wherein the at least two apertures are configured to engage with the gripping mechanism. In some embodiments, the at least one aperture may be positioned at an upper surface of the cartridge. In some embodiments, the cartridge comprises the scannable identification mechanism. In some embodiments, the scannable identification mechanism of the cartridge comprises the RFID.

Described herein are various methods comprising: providing or obtaining the system, in accordance with some embodiments, and producing and/or synthesizing a nucleic acid. In some embodiments, the nucleic acid is a ribonucleic acid (RNA).

### Compounds

Provided herein is a system for producing a compound, including drug substance, active pharmaceutical ingredient and/or biopharmaceutical compounds, comprising a plurality of chambers, at least one robotic arm and at least one computer processor operatively coupled to the plurality of chambers and the at least one robotic arm. In some embodiments, each chamber of the plurality of chambers is configured to contain at least one reagent for producing the compound. In further embodiments, the at least one robotic arm is configured to load each chamber with the at least one reagent.

In some embodiments described herein, the at least one robotic arm is programmable to (i) fill a first chamber with the at least one reagent; (ii) fill a second chamber after a time interval X with the at least one reagent; and (iii) repeat step (ii) until at least a portion of the plurality of chambers is filled with the at least one reagent. In some embodiments, the compound is produced and removed after a time interval Y from each of the portions of plurality of chambers in step (iii). In some embodiments, the system is programmable in at least a portion of the plurality of chambers to re-load, produce and remove the compound as in steps (i)-(iii) thereby for a time period of at least 2Y continuously producing the compound within at least a portion of the plurality of the chambers.

In some embodiments, the compound is continuously produced for a time period of at least 3Y within at least a portion of the plurality of chambers. In yet other embodiments, the compound is continuously produced for a time period of at least 4Y within at least a portion of the plurality of chambers. In still other embodiments, the compound is continuously produced for a time period of at least 5Y within at least a portion of the plurality of chambers. In still other embodiments, the compound is continuously produced for a time period of at least more than 5Y within at least a portion of the plurality of chambers. In some embodiments, the compound is continuously produced for a time period of at least 6Y within at least a portion of the plurality of chambers. In some embodiments, the compound is continuously produced for a time period of at least 7Y within at least a portion of the plurality of chambers. In some embodiments, the compound is continuously produced for a time period of at least 8Y within at least a portion of the plurality of chambers. In some embodiments, the compound is continuously produced for a time period of at least 9Y within at least a portion of the plurality of chambers. In some embodiments, the compound is continuously produced for a time period of at least 10Y within at least a portion of the plurality of chambers. In some embodiments, the compound is continuously produced for a time period of at least 20Y within at least a portion of the plurality of chambers. In some embodiments, the compound is continuously produced for a time period of at least 30Y within at least a portion of the plurality of chambers. In some embodiments, the compound is continuously produced for a time period of at least 40Y within at least a portion of the plurality of chambers. In some embodiments, the compound is continuously produced for a time period of at least 50Y within at least a portion of the plurality of chambers. In some embodiments, the compound is continuously produced for a time period of at least 60Y within at least a portion of the plurality of chambers. In some embodiments, the compound is continuously produced for a time period of at least 70Y within at least a portion of the plurality of chambers. In some embodiments, the compound is continuously produced for a time period of at least 80Y within at least a portion of the plurality of chambers. In some embodiments, the compound is continuously produced for a time period of at least 90Y within at least a portion of the plurality of chambers. In some embodiments, the compound is continuously produced for a time period of at least 100Y within at least a portion of the plurality of chambers. In some embodiments, the compound is continuously produced for a time period of at least 200Y within at least a portion of the plurality of chambers. In some embodiments, the compound is continuously produced for a time period of at least 300Y within at least a portion of the plurality of chambers. In some embodiments, the compound is continuously produced for a time period of at least 400Y within at least a portion of the plurality of chambers. In some embodiments, the compound is continuously produced for a time period of at least 500Y within at least a portion of the plurality of chambers.

In some embodiments described herein, the compound, including biopharmaceutical compounds, comprises nucleic acids including DNA and RNA, modified DNA and RNA, polypeptides, proteins, and modified proteins.

In some embodiments, the biochemical compound comprises RNA molecules that can be used in current and/or potential RNA-based therapies. In some embodiments, the biochemical compound comprises long-chain RNA, coding RNA, non-coding RNA, long non-coding RNA, single stranded RNA (ssRNA), double stranded RNA (dsRNA), linear RNA (linRNA), circular RNA (circRNA), messenger RNA (mRNA), self-amplifying mRNA (SAM), Trans amplifying mRNA, RNA oligonucleotides, antisense oligonucleotides, small interfering RNA (siRNA), small hairpin RNA (shRNA), antisense RNA (asRNA), CRISPR/Cas9 guide RNAs, riboswitches, immunostimulating RNA (isRNA), ribozymes, aptamers, ribosomal RNA (rRNA), transfer RNA (tRNA), viral RNA (vRNA), retroviral RNA or replicon RNA, small nuclear RNA (snRNA), small nucleolar RNA (snoRNA), microRNA (miRNA), and a Piwi-interacting RNA (piRNA).

In some embodiments, the biochemical compound comprises modified RNA molecules. In some embodiments, the modification of RNA molecule comprises chemical modifications comprising backbone modifications as well as sugar modifications or base modifications. In this context, a modified RNA molecule as defined herein comprises nucleotide analogues/modifications, e.g. backbone modifications, sugar modifications or base modifications. A backbone modification in connection with the present disclosure is a modification, in which phosphates of the backbone of the nucleotides contained in an RNA molecule are chemically modified. A sugar modification in connection with the present disclosure is a chemical modification of the sugar of the nucleotides of the RNA molecule. Furthermore, a base modification in connection with the present disclosure is a chemical modification of the base moiety of the nucleotides of the RNA molecule. In this context, nucleotide analogues or modifications are selected from nucleotide analogues, which are applicable for transcription and/or translation. In further embodiments, the modified RNA comprises nucleoside modifications selected from 6-aza-cytidine, 2-thio-cytidine, α-thio-cytidine, pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-chloro-purine, N6-methyl-2-amino-purine, pseudo-iso-cytidine, 6-chloro-purine, N6-methyl-adenosine, α-thio-adenosine, 8-azido-adenosine, 7-deaza-adenosine.

In some embodiments, the biochemical compound comprises protein molecules that can be used in current and/or potential protein-based therapies. In some embodiments, the biochemical compound comprises antibody-based drugs, glycoconjugates, Fc fusion proteins, anticoagulants, blood factors, bone morphogenetic proteins, engineered protein scaffolds, enzymes, growth factors, hormones, interferons, interleukins and other cytokines, viral (and other pathogen) proteins (or antigens), parts of proteins (truncated proteins, ectodomains, stem domains, etc.) or chimeric proteins and thrombolytics. In some embodiments, the biochemical compound comprises modified protein molecules that can be used in protein-based therapies. In some embodiments, the modification of protein molecule comprises phosphorylation, glycosylation, acetylation, ubiquitylation/sumoylation, methylation, palmitoylation, quinone, amidation, myristoylation, pyrrolidone carboxylic acid, hydroxylation, phosphopantetheine, prenylation, GPI anchoring, oxidation, ADP-ribosylation, sulfation, S-nitrosylation, citrullination, nitration, gamma-carboxyglutamic acid, formylation, hypusine, topaquinone /TPQ, bromination, lysine topaquinone/LTQ, tryptophan tryptophylquinone/TTQ, iodination, pegylation and cysteine tryptophylquinone / CTQ.

### Compound production

In some embodiments of the present disclosure, the chamber is designed to accommodate a reaction/process or part of a reaction/process taking place in the chamber. In some embodiments, the reaction relates to in vitro transcription of RNA from a DNA template and/or in vitro (cell-free) translation of RNA to protein and/or a combination of both processes, i.e., from DNA to RNA through transcription and from RNA to protein through translation. In some embodiments, the reaction may also pertain to the culturing and growing of cells and expressing proteins or viral particles. Not meant to be limiting, in further embodiments, the reaction may also relate to any incubation steps following a given temperature and time profile.

In some embodiments, the chamber is configured to conduct an in vitro transcription reaction. In some embodiments, the chamber is configured to conduct an in vitro translation. In some embodiments, the in vitro translation is cell dependent. In some embodiments, the in vitro translation is cell free. In some embodiments, the chamber is not designed to culture a living cell.

In some embodiments, the in vitro transcription relates to a process in which RNA is synthesized in a cell-free system (in vitro). In some embodiments, cloning vector(s) DNA, particularly plasmid DNA vectors are applied as template for the generation of RNA transcripts following linearization of circular plasmid DNA molecule. These cloning vectors are generally designated as transcription vector. RNA may be obtained by DNA dependent in vitro transcription of an appropriate DNA template. A promoter for controlling RNA in vitro transcription can be any promoter for any DNA dependent RNA polymerase. In some embodiments, a viral promoter binds a viral RNA polymerase and is at least one promoter selected from the list consisting of T7, T3, T7lac, SP6, pL, CMV, SV40, and CaMV35S. Alternately or in combination, the nucleic acid fragment comprising promoter sequence comprises a bacterial promoter. In some embodiments, the bacterial promoter binds a bacterial RNA polymerase and is at least one promoter selected from the list consisting of araBAD, trp, lac, and Ptac. Sometimes, the nucleic acid fragment comprising promoter sequence comprises a eukaryotic promoter. In some embodiments, the eukaryotic promoter binds a eukaryotic RNA polymerase and is at least one promoter selected from the list consisting of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. In some embodiments, the eukaryotic promoter is at least one promoter selected from the list consisting of an RNA pol I promoter, an RNA pol II promoter and an RNA pol III promoter.

In some embodiments, the DNA dependent RNA polymerases comprise at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. The DNA template for RNA in vitro RNA transcription may be obtained by cloning of a nucleic acid, in particular cDNA corresponding to the respective RNA to be in vitro transcribed, and introducing it into an appropriate vector for RNA in vitro transcription, for example in plasmid circular plasmid DNA. The cDNA may be obtained by reverse transcription of mRNA or chemical synthesis. Moreover, the DNA template for in vitro RNA synthesis may also be obtained by gene synthesis.

In some embodiments, the DNA template relates to a DNA molecule comprising a nucleic acid sequence encoding the RNA sequence. The template DNA is used as a template for RNA in vitro transcription in order to produce the RNA encoded by the template DNA. Therefore, the template DNA comprises all elements necessary for RNA in vitro transcription, particularly a promoter element for binding of a DNA dependent RNA polymerase as e.g. T3, T7 and SP6 RNA polymerases 5' of the DNA sequence encoding the target RNA sequence. The poly(A) tail can be either encoded into the DNA template or added enzymatically to RNA in a separate step after in vitro transcription. In some embodiments, the template DNA comprises primer binding sites 5' and/or 3' of the DNA sequence encoding the target RNA sequence to determine the identity of the DNA sequence encoding the target RNA sequence e.g. by PCR or DNA sequencing. In some embodiments, the DNA template comprises a DNA vector, such as a plasmid DNA, which comprises a nucleic acid sequence encoding the RNA sequence. In some embodiments, the DNA template comprises a linear or a circular DNA molecule.

In some embodiments of the present invention, a DNA template encodes a different RNA molecule species. In some embodiments the DNA template contains a sub-genomic promoter and a large ORF encoding for non-structural proteins which, following delivery of the biopharmaceutical into the cytosol, are transcribed in four functional components (nsP1, nsP2, nsP3, and nsp4) by the encoded RNA-dependent RNA polymerase (RDRP). RDRP than produces a negative-sense copy of the genome which serves as a template for two positive-strand RNA molecules: the genomic mRNA and a shorter sub-genomic mRNA. This sub-genomic mRNA is transcribed at very high levels, allowing the amplification of mRNA encoding the antigen of choice. A different RNA molecule species may encode an antigen of different serotypes or strains of a pathogen, a different allergen, a different autoimmune antigen, a different antigen of a pathogen, different adjuvant proteins, a different isoform or variant of a cancer or tumor antigen, a different tumor antigen of one patient, one antibody among a group of antibodies which target different epitopes of a protein or of a group of proteins, different proteins of a metabolic pathway, a single protein among a group of proteins which are defect in a subject, or a different isoform of a protein for molecular therapy.

In some embodiments, the pathogen is selected from the group consisting of a virus, bacterium, prion, fungus, protozoon, viroid, and parasite.

In some embodiments, the pathogen is selected from the group that causes human disease which includes but are not limited to, Bacillus anthracis (anthrax), Clostridium botulinum toxin (botulism), Yersinia pestis (plague). Variola major (smallpox) and other related pox viruses, Francisella tularensis (tularemia), Viral hemorrhagic fevers, Arenaviruses, (e.g., Junin, Machupo, Guanarito, Chapare, Lassa, and/or Lujo), Bunyaviruses (e.g., Hantaviruses causing Hanta Pulmonary syndrome, Rift Valley Fever, and/or Crimean Congo Hemorrhagic Fever), Flaviviruses, Dengue, Filoviruses (e.g., Ebola and Marburg viruses), Burkholderia pseudomallei (melioidosis), Coxiella burnetii (Q fever), Brucella species (brucellosis), Burkholderia mallei (glanders), Chlamydia psittaci (Psittacosis), Ricin toxin (Ricinus communis), Epsilon toxin (Clostridium perfringens), Staphylococcus enterotoxin B (SEB), Typhus fever (Rickettsia prowazekii), Food- and waterborne pathogens, Diarrheagenic E . coli, Pathogenic Vibrios, Shigella species, Salmonella, Listeria monocytogenes, Campylobacter jejuni, Yersinia enterocolitica, Caliciviruses, Hepatitis A, Cryptosporidium parvum, Cyclospora cayatanensis, Giardia lamblia, Entamoeba histolytica , Toxoplasma gondii, Naegleria fowleri, Balamuthia mandrillaris, Fungi, Microsporidia, Mosquito-borne viruses (e.g., West Nile virus (WNV), LaCrosse encephalitis (LACV), California encephalitis, Venezuelan equine encephalitis (VEE), Eastern equine encephalitis (EEE), Western equine encephalitis (WEE), Japanese encephalitis virus (JE), St. Louis encephalitis virus (SLEV), Yellow fever virus (YFV), Chikungunya virus, Zika virus, Nipah and Hendra viruses, Additional hantaviruses, Tickborne hemorrhagic fever viruses, Bunyaviruses, Severe Fever with Thrombocytopenia Syndrome virus (SFTSV), Heartland virus, Flaviviruses (e.g., Omsk Hemorrhagic Fever virus, Alkhurma virus, Kyasanur Forest virus), Tickborne encephalitis complex flaviviruses, Tickborne encephalitis viruses, Powassan/Deer Tick virus, Tuberculosis, including drug-resistant Tuberculosis, Influenza virus, Prions, Streptococcus, Pseudomonas, Shigella, Campylobacter, Salmonella, Clostridium, Escherichia, Hepatitis C, papillomavirus, Epstein-Barr virus, varicella, variola, Orthomyxovirus, Severe acute respiratory syndrome associated coronavirus (SARS-CoV), SARS-CoV-2 (COVID-19), MERS-CoV, other highly pathogenic human coronaviruses, or any combination thereof.

In some embodiments, the virus is a respiratory virus that primarily results in respiratory symptoms including, without limitation, coronaviruses, influenza viruses, adenoviruses, rhinoviruses, coxsackieviruses, and metapneumoviruseses. In some embodiments, the virus is an enteric virus that primarily results in digestive symptoms including, without limitation, enteroviruses, noroviruses, heptoviruses, reoviruses, rotaviruses, parvoviruses, toroviruses, and mastadenovirus. In certain embodiments, the virus is a hemorrhagic fever virus including, without limitation, Ebola virus, Marburg virus, dengue fever virus, yellow fever virus, Rift valley fever virus, hanta virus, and Lassa fever virus.

In some embodiments, a DNA template encodes a different RNA molecule species. In some embodiments, different RNA molecule species comprises RNA constructs representing an engineered (non-natural) variant of a protein (e.g., chimeric protein), or fragment thereof. In some embodiments, a DNA template encodes different whole proteins that are separated by a linker. In some embodiments, different RNA molecule species encodes a different pathogenic antigen or a fragment or variant thereof. In some embodiments, the pathogen-associated antigen is from an influenza virus. In some embodiments, the pathogen-associated antigen is from an influenza A virus, such as the H5N1 strain. In some embodiments, the pathogen-associated antigen is from an influenza B virus. In some embodiments, the pathogen-associated antigen is an influenza matrix M1 protein or a fragment thereof. In certain embodiments, the pathogen-associated antigen is an influenza neuraminidase or a fragment thereof. In certain embodiments, the pathogen-associated antigen is an influenza hemagglutinin or a fragment thereof. For example, the pathogen-associated antigen may comprise an entire hemagglutinin, an HA1 domain, an HA2 domain or any antigenic portion thereof.

In some embodiments, the pathogen-associated antigen is a Coronaviridae antigen. In some embodiments, the Coronaviridae exhibits human tropism. In some embodiments, the Coronaviridae is selected from the list consisting of SARS Coronavirus (SARS-CoV-1), COVID-19 (SARS-CoV-2), MERS-coronavirus (MERS-CoV), or any combination thereof. In some embodiments, the Coronaviridae comprises SARS Coronavirus (SARS-CoV-1). In some embodiments, the Coronaviridae comprises COVID-19 (SARS-CoV-2). In some embodiments, the Coronaviridae comprises MERS-coronavirus (MERS-CoV). In some embodiments, the Coronaviridae antigen comprises a spike protein, an envelope protein, a nucleocapsid protein, a membrane protein, a membrane glycoprotein, or a non-structural protein. In some embodiments, the Coronaviridae antigen comprises a spike protein, an envelope small membrane protein, a membrane protein, a non-structural protein 6 (NSP6), a nucleoprotein, an ORF10 protein, Protein 3a, Protein7a, Protein 9b, structural protein 8, uncharacterized protein 4, or any combination thereof.

In some embodiments, a DNA template encodes a different RNA molecule species. In some embodiments, a different RNA molecule species encodes a different cancer or tumor antigen, or a fragment or variant thereof, selected from the group consisting of caTRL4, CD40L, CD34, CD41, G6B, P-selectin, Clec2, cKIT, FLT3, MPL, ITGB3, ITGB2, GP5, GP6, GP9, GP1BA, DSC2, FCGR2A, TNFRSF10A, TNFRSF10B, TM4SF1, Her2, Trop2, CEA, NaPi2b, uPAR, CDCP1, MUC-1, MUC-16, CEACAM-5, MR-1, Fn14, MAGE-3, NY-ESO-1, EGFR, PDGFR, IGF1R, CSF-1R, PSMA, PSCA, STEAP-1, FAP, TEM8, 5T4, VEGFR, NRP1, CD19, CD20, CD22, CD25, CD30, CD33, CD37, CD38, CD39, CD44, CD47, CD52, CD70, CD71, CD74, CD79b, CD132, CD133, CD138, CD166, CD205, CD276, ROR1, ROR2, Glypican 3, Trail Receptor 2 (DR5), PD-L1, Mesothein, Bombesin, EpCAM, DARPP, CSPG4, Galectin-3, Integrin αvβ1, Integrin αvβ3, Integrin αvβ5, Integrin αvβ6, Integrin α5β1, Integrin alpha-3, Integrin alpha-5, Integrin beta-6, Nectin-4, DLL3, Transferrin Receptor, Folate Receptor alpha, Tissue Factor, BCMA, c-Met, LIV-1, AXL, AFP, ENPP3, CLDN6/9, DPEP3, RNF43, LRRC15, PTK7, P-cadherin, FLT3, EphA2, MTI-MMP, CXCR6, GD2, or Smoothened antigen (Smo).

### Components of the System

Described herein are various embodiments, of a system comprising a chamber; a cartridge; a separation and/or purification device; a lid; a sensor; an identification (ID) mechanism; or a combination thereof.

### Chambers & Cartridge

Described herein are various embodiments of a chamber for the production of a compound, the chamber comprising a bottom portion, a body portion comprising an inner volume configured to hold at least one reagent or a compound, an open end configured to receive at least one reagent, wherein at least part of said body portion comprises a flat polygonal surface configured to position and/or support a sensor in the reaction chamber. In some embodiments, the body portion comprises at least three flat polygonal surfaces. In some embodiments, a cross-section of the body portion is polygonal shape. In some embodiments, polygonal shape comprises a trigonal, tetragonal, pentagonal or hexagonal shape. In some embodiments, the cross-section of the body portion is hexagonal. In some embodiments, the open end is configured to receive a removable lid for at least partially closing the open end. In some embodiments, the bottom is rounded. In some embodiments, the chamber comprises a chamber volume of about 0.1 mL to about 1000 mL. In some embodiments, the chamber is configured for single use or multiple uses. The chamber of any one of the preceding claims, further comprising a scannable identification (ID) mechanism. In some embodiments, the scannable ID mechanism comprises an RFID.

Described herein are various embodiments of an assembly comprising a lid configured to at least partially close the open end of the chamber. In some embodiments, the lid comprises a removable lid. In some embodiments, the lid comprises a push-on lid. In some embodiments, the lid comprises a screw lid. In some embodiments, the lid comprises a non-removable lid. In some embodiments, the lid is bonded to at least a part of the chamber. In some embodiments, the lid is adhered to at least a part of the chamber. In some embodiments, the lid is glued to at least part of the chamber. In some embodiments, the lid comprises an opening. In some embodiments, the opening is centrally located within the lid. In some embodiments, the lid comprises a puncturable membrane. In some embodiments, the puncturable membrane comprises an elastic membrane.

Described herein are various embodiments of a cartridge comprising a plurality of chambers. In some embodiments, the at least one aperture comprises between 2 and 20 chambers. In some embodiments, the at least one aperture comprises between 2 and 10 chambers. In some embodiments, the cartridge further comprises at least one aperture. In some embodiments, the at least one aperture is configured to engage with a gripping mechanism. In some embodiments, the cartridge is configured to engage with a robotic arm and/or a handling device. In some embodiments, the robotic arm and/or the handling device comprises the gripping mechanism. In some embodiments, the cartridge comprises at least two apertures, wherein the at least two apertures are configured to engage with the gripping mechanism. In some embodiments, the at least one aperture is positioned at an upper surface of the cartridge. The cartridge of any one of the preceding claims, further comprising the scannable ID mechanism. In some embodiments, the scannable ID mechanism comprises the RFID.

In some embodiments, the plurality of chambers is housed in a cartridge.

In some embodiments, the cartridge comprises at least 2 chambers, 3 chambers, 4 chambers, 5 chambers, 6 chambers, 7 chambers, 8 chambers, 9 chambers, 10 chambers, 11 chambers, 12 chambers, 13 chambers, 14 chambers, 15 chambers, 16 chambers, 17 chambers, 18 chambers, 19 chambers, 20 chambers, 21 chambers, 22 chambers, 23 chambers, 24 chambers, 25 chambers, 26 chambers, 27 chambers, 28 chambers, 29 chambers, 30 chambers, 31 chambers, 32 chambers, 33 chambers, 34 chambers, 35 chambers, 36 chambers, 37 chambers, 38 chambers, 39 chambers, 40 chambers, 41 chambers, 42 chambers, 43 chambers, 44 chambers, 45 chambers, 46 chambers, 47 chambers, 48 chambers, 49 chambers, or 50 chambers. In some embodiments, the cartridge comprises at least about 60 chambers, about 70 chambers, about 80 chambers, about 90 chambers, about 100 chambers, about 110 chambers, about 120 chambers, about 130 chambers, about 140 chambers, about 150 chambers, about 160 chambers, about 170 chambers, about 180 chambers, about 190 chambers, or about 200 chambers.

In some embodiments, the cartridge comprises 2 to 20 chambers. In some embodiments, the cartridge comprises 2 to 10 chambers. In some embodiments, the cartridge comprises at least about 2 chambers. In some embodiments, the cartridge comprises at least about 12 chambers. In some embodiments, the cartridge comprises at least about 24 chambers. In some embodiments, the cartridge comprises at least about 36 chambers. In some embodiments, the cartridge comprises at least about 48 chambers.
In some embodiments, the chamber is removable from the cartridge. In some embodiments, the chamber is not removable from the cartridge.

In some embodiments, the chamber comprises a volume of at least about 0.1 mL to 1000 mL. In some embodiments, the chamber comprises a volume of at least about 0.1 mL, about 0.3 mL, about 0.5 mL, about 1 mL, about 1.5 mL, about 2 mL, about 2.5 mL, about 3 mL, about 4 mL, about 5 mL, about 6 mL, about 7 mL, about 8 mL, about 9 mL, about 10 mL, about 12 mL, about 15 mL, about 17 mL, about 20 mL, about 25 mL, about 30 mL, about 35 mL, about 40 mL, about 45 mL, about 50 mL, about 55 mL, about 60 mL, about 65 mL, about 70 mL, about 75 mL, about 80 mL, about 85 mL, about 90 mL, about 95 mL, about 100 mL, about 300 mL, about 500 mL, about 1000 mL, or more. In some embodiments, the chamber comprises a volume of not more than about 1000 mL, not more than about 500 mL, not more than about 300 mL, not more than about100 mL, not more than about 95 mL, not more than about 90 mL, not more than about 85 mL, not more than about 80 mL, not more than about 75 mL, not more than about 70 mL, not more than about 65 mL, not more than about 60 mL, not more than about 55 mL, not more than about 50 mL, not more than about 45 mL, not more than about 40 mL, not more than about 35 mL, not more than about 30 mL, not more than about 25 mL, not more than about 20 mL, not more than about 15 mL, not more than about 10 mL, not more than about 9 mL, not more than about 8 mL, not more than about 7 mL, not more than about 6 mL, not more than about 5 mL, not more than about 4 mL, not more than about 3 mL, not more than about 2 mL, not more than about 1 mL, not more than about 0.5 mL, not more than about 0.3 mL, or not more than about 0.1 mL. In some embodiments, the chamber comprises a volume of between about 1 mL to about 100 mL, between about 10 mL to 90 mL, between about 15 mL to about 80 mL, between about 20 mL to about 70 mL, between about 25 mL to about 60 mL or between about 30 mL to about 50 mL.

In some embodiments, the chamber comprises a volume of at least about 150 mL, about 200 mL, about 250 mL, about 300 mL, about 350 mL, about 400 mL, about 450 mL, about 500 mL, about 550 mL, about 600 mL, about 650 mL, about 700 mL, about 750 mL, about 800 mL, about 850 mL, about 900 mL, about 950 mL, about 1000 mL, about 2000 mL, about 3000 mL, about 4000 mL, about 5000 mL, about 6000 mL, about 7000 mL, about 8000 mL, about 9000 mL, about 10000 mL, about 15000 mL, about 20000 mL, about 25000 mL, about 30000 mL, about 35000 mL, about 40000 mL, about 45000 mL, or about 50000 mL. In some embodiments, the chamber comprises a volume of not more than about 50000 mL, not more than about 45000 mL, not more than about 40000 mL, not more than about 35000 mL, not more than about 30000 mL, not more than about 25000 mL, not more than about 20000 mL, not more than about 15000 mL, not more than about 10000 mL, not more than about 9000 mL, not more than about 8000 mL, not more than about 7000 mL, not more than about 6000 mL, not more than about 5000 mL, not more than about 4000 mL, not more than about 3000 mL, not more than about 2000 mL, not more than about 1000 mL, not more than about 950 mL, not more than about 900 mL, not more than about 850 mL, not more than about 800 mL, not more than about 750 mL, not more than about 700 mL, not more than about 650 mL, not more than about 600 mL, not more than about 550 mL, not more than about 500 mL, not more than about 450 mL, not more than about 400 mL, not more than about 350 mL, not more than about 300 mL, not more than about 250 mL, not more than about 200 mL, not more than about 150 mL. In some embodiments, the chamber comprises a volume of between about 150 mL to about 50000 mL, between about 200 mL to 45000 mL, between about 250 mL to about 40000 mL, between about 300 mL to about 35000 mL, between about 350 mL to about 30000 mL, between about 400 mL to about 25000 mL, between about 450 mL to about 20000 mL, between about 500 mL to about 15000 mL. between about 550 mL to about 10000 mL, between about 600 mL to about 9000 mL, between about 650 mL to 8000 mL, between about 700 mL to about 7000 mL, between about 750 mL to about 6000 mL, between about 800 mL to about 5000 mL, between about 850 mL to about 4000 mL, between about 900 mL to about 3000 mL, between about 950 mL to about 2000 mL or between about 1000 mL to about 1500 mL.

In some embodiments described herein, a number of the plurality of chambers is at least the time interval Y divided by the time interval X. In some embodiments, a number of the plurality of chambers is less than the time interval Y divided by the time interval X. In some embodiments, a number of the plurality of chambers is greater than the time interval Y divided by the time interval X. In some embodiments, when the last chamber of the plurality of chambers is filled with the at least one reagent, the compound is produced and removed from the first chamber of the plurality of chambers. In some embodiments, the time interval Y is at least the time interval X times the number of the plurality of chambers. In some embodiments, for a time period of at least 2Y, the system continuously produces the compound within the plurality of the chambers. In some embodiments, the compound is continuously produced for a time period of at least 3Y within at least a portion of the plurality of chambers. In yet other embodiments, the compound is continuously produced for a time period of at least 4Y within at least a portion of the plurality of chambers. In still other embodiments, the compound is continuously produced for a time period of at least 5Y within at least a portion of the plurality of chambers. In still other embodiments, the compound is continuously produced for a time period of at least 6Y within at least a portion of the plurality of chambers. In still other embodiments, the compound is continuously produced for a time period of at least 7Y within at least a portion of the plurality of chambers. In still other embodiments, the compound is continuously produced for a time period of at least 8Y within at least a portion of the plurality of chambers. In still other embodiments, the compound is continuously produced for a time period of at least 9Y within at least a portion of the plurality of chambers. In still other embodiments, the compound is continuously produced for a time period of at least 10Y within at least a portion of the plurality of chambers. In still other embodiments, the compound is continuously produced for a time period of at least 20Y within at least a portion of the plurality of chambers. In still other embodiments, the compound is continuously produced for a time period of at least 30Y within at least a portion of the plurality of chambers. In still other embodiments, the compound is continuously produced for a time period of at least 40Y within at least a portion of the plurality of chambers. In still other embodiments, the compound is continuously produced for a time period of at least 50Y within at least a portion of the plurality of chambers. In still other embodiments, the compound is continuously produced for a time period of at least 60Y within at least a portion of the plurality of chambers. In still other embodiments, the compound is continuously produced for a time period of at least 70Y within at least a portion of the plurality of chambers. In still other embodiments, the compound is continuously produced for a time period of at least 80Y within at least a portion of the plurality of chambers. In still other embodiments, the compound is continuously produced for a time period of at least 90Y within at least a portion of the plurality of chambers. In still other embodiments, the compound is continuously produced for a time period of at least 100Y within at least a portion of the plurality of chambers. In still other embodiments, the compound is continuously produced for a time period of at least 200Y within at least a portion of the plurality of chambers. In still other embodiments, the compound is continuously produced for a time period of at least 300Y within at least a portion of the plurality of chambers. In still other embodiments, the compound is continuously produced for a time period of at least 400Y within at least a portion of the plurality of chambers. In still other embodiments, the compound is continuously produced for a time period of at least 500Y within at least a portion of the plurality of chambers. In still other embodiments, the compound is continuously produced for a time period of at least more than 5Y within at least a portion of the plurality of chambers.

In some embodiments, the time interval X comprises time at least about 0.1 minute, about 0.2 minutes, about 0.3 minutes, about 0.4 minutes, about 0.5 minutes, about 0.6 minutes, about 0.7 minutes, about 0.8 minutes, about 0.9 minutes, about 1 minute, about 1.1 minutes, about 1.2 minutes, about 1.3 minutes, about 1.4 minutes, about 1.5 minutes, about 1.6 minutes, about 1.7 minutes, about 1.8 minutes, about 1.9 minutes, about 2 minutes, about 2.1 minutes, about 2.2 minutes, about 2.3 minutes, about 2.4 minutes, about 2.5 minutes, about 2.6 minutes, about 2.7 minutes, about 2.8 minutes, about 2.9 minutes, about 3 minutes, about 3.1 minutes, about 3.2 minutes, about 3.3 minutes, about 3.4 minutes, about 3.5 minutes, about 3.6 minutes, about 3.7 minutes, about 3.8 minutes, about 3.9 minutes, about 4 minutes, about 4.1 minutes, about 4.2 minutes, about 4.3 minutes, about 4.4 minutes, about 4.5 minutes, about 4.6 minutes, about 4.7 minutes, about 4.8 minutes, about 4.9 minutes, about 5 minutes, about 5.1 minutes, about 5.2 minutes, about 5.3 minutes, about 5.4 minutes, about 5.5 minutes, about 5.6 minutes, about 5.7 minutes, about 5.8 minutes, about 5.9 minutes, about 6 minutes, about 6.1 minutes, about 6.2 minutes, about 6.3 minutes, about 6.4 minutes, about 6.5 minutes, about 6.6 minutes, about 6.7 minutes, about 6.8 minutes, about 6.9 minutes, about 7 minutes, about 7.1 minutes, about 7.2 minutes, about 7.3 minutes, about 7.4 minutes, about 7.5 minutes, about 7.6 minutes, about 7.7 minutes, about 7.8 minutes, about 7.9 minutes, about 8 minutes, about 8.1 minutes, about 8.2 minutes, about 8.3 minutes, about 8.4 minutes, about 8.5 minutes, about 8.6 minutes, about 8.7 minutes, about 8.8 minutes, about 8.9 minutes, about 9 minutes, about 9.1 minutes, about 9.2 minutes, about 9.3 minutes, about 9.4 minutes, about 9.5 minutes, about 9.6 minutes, about 9.7 minutes, about 9.8 minutes, about 9.9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 22 minutes, about 24 minutes, about 26 minutes, about 28 minutes, about 30 minutes, about 35 minutes, about 40 minutes, about 45 minutes, about 50 minutes, about 55 minutes, or about 60 minutes. In some embodiments, the time interval X comprises time not more than about 60 minutes, not more than about 55 minutes, not more than about 50 minutes, not more than about 45 minutes, not more than about 35 minutes, not more than about 30 minutes, not more than about 25 minutes, not more than about 20 minutes, not more than about 15 minutes, not more than about 10 minutes, not more than about 5 minutes, not more than about 4 minutes, not more than about 3 minutes, not more than about 2 minutes, not more than about 1 minute, not more than about 0.5 minutes or not more than about 0.1 minute. In some embodiments, the time interval X comprises time between about 60 minutes to about 0.1 minute, between about 55 minutes to about 0.5 minute, between about 50 minutes to about 1 minute, between about 45 minutes to about 2 minute, between about 40 minutes to about 3 minutes, between about 35 minutes to about 4 minutes, between about 30 minutes to about 5 minutes, between about 25 minutes to about 10 minutes or between about 20 minutes to about 15 minutes.

In some embodiments, the time interval X comprises time at least about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, or about 36 hours. In some embodiments, the time interval X comprises time not more than about 36 hours, not more than about 35 hours, not more than about 34 hours, not more than about 33 hours, not more than about 32 hours, not more than about 31 hours, not more than about 30 hours, not more than about 29 hours, not more than about 28 hours, not more than about 27 hours, not more than about 26 hours, not more than about 25 hours, not more than about 24 hours, not more than about 23 hours, not more than about 22 hours, not more than about 21 hours, not more than about 20 hours, not more than about 19 hours, not more than about 18 hours, not more than about 17 hours, not more than about 16 hours, not more than about 15 hours, not more than about 14 hours, not more than about 13 hours, not more than about 12 hours, not more than about 11 hours, not more than about 10 hours, not more than about 9 hours, not more than about 8 hours, not more than about 7 hours, not more than about 6 hours, not more than about 5 hours, not more than about 4 hours, not more than about 3 hours, not more than about 2 hours or not more than about 1 hour. In some embodiments, the time interval X comprises time between about 36 hours to about 1 hour, between about 35 hours to about 2 hours, between about 34 hours to about 3 hours, between about 33 hours to about 4 hours, between about 32 hours to about 5 hours, between about 31 hours to about 6 hours, between about 30 hours to about 7 hours, between about 29 hours to about 8 hours, between about 28 hours to about 9 hours, between about 27 hours to about 10 hours, between about 26 hours to about 11 hours, between about 25 hours to about 12 hours, between about 24 hours to about 13 hours, between about 23 hours to about 14 hours, between about 22 hours to about 15 hours, between about 21 hours to about 16 hours, between about 20 hours to about 17 hours or between about 19 hours to about 18 hours.

In some embodiments, the time interval Y comprises at least about 10 minutes, about 20 minutes, about 30 minutes, about 40 minutes, about 50 minutes, about 60 minutes, about 65 minutes, about 70 minutes, about 75 minutes, about 80 minutes, about 85 minutes, about 90 minutes, about 95 minutes, about 100 minutes, about 150 minutes, about 200 minutes, about 250 minutes, about 300 minutes, about 350 minutes, about 400 minutes, about 450 minutes, about 500 minutes, about 550 minutes, about 600 minutes, about 650 minutes, about 700 minutes, about 750 minutes, about 800 minutes, about 850 minutes, about 900 minutes, about 950 minutes, about 1000 minutes, about 1100 minutes, about 1200 minutes, about 1300 minutes, about 1400 minutes, about 1500 minutes, about 1600 minutes, about 1700 minutes, about 1800 minutes, about 1900 minutes, about 2000 minutes, about 2100 minutes, about 2200 minutes, about 2300 minutes, or about 2400 minutes. In some embodiments, the time interval Y comprises time not more than about 2400 minutes, not more than about 2300 minutes, not more than about 2200 minutes, not more than about 2100 minutes, not more than about 2000 minutes, not more than about 1900 minutes, not more than about 1800 minutes, not more than about 1700 minutes, not more than about 1600 minutes, not more than about 1500 minutes, not more than about 1400 minutes, not more than about 1300 minutes, not more than about 1200 minutes, not more than about 1100 minutes, not more than about 1000 minutes, not more than about 950 minutes, not more than about 900 minutes, not more than about 850 minutes, not more than about 800 minutes, not more than about 750 minutes, not more than about 700 minutes, not more than about 650 minutes, not more than about 600 minutes, not more than about 550 minutes, not more than about 500 minutes, not more than about 450 minutes, not more than about 400 minutes, not more than about 350 minutes, not more than about 300 minutes, not more than about 250 minutes, not more than about 200 minutes, not more than about 150 minutes, not more than about 100 minutes, not more than about 95 minutes, not more than about 90 minutes, not more than about 85 minutes, not more than about 80 minutes, not more than about 75 minutes, not more than about 70 minutes, not more than about 65 minutes, not more than about 60 minutes, not more than about 55 minutes, not more than about 50 minutes, not more than about 45 minutes, not more than about 40 minutes, not more than about 35 minutes, not more than about 30 minutes, not more than about 25 minutes, not more than about 20 minutes, not more than about 15 minutes or not more than about 10 minutes. In some embodiments, the time interval Y comprises time between about 2400 minutes to about 10 minutes, between about 2300 minutes to about 20 minutes, between about 2200 minutes to about 30 minutes, between about 2100 minutes to about 40 minutes, between about 2000 minutes to about 50 minutes, between about 1900 minutes to about 60 minutes, between about 1800 minutes to about 70 minutes, between about 1700 minutes to about 80 minutes, between about 1600 minutes to about 90 minute, between about 1500 minutes to about 100 minutes, between about 1400 minutes to about 150 minutes, between about 1300 minutes to about 200 minutes, between about 1200 minutes to about 250 minutes, between about 1100 minutes to about 300 minutes, between about 1000 minutes to about 350 minutes, between about 950 minutes to about 400 minutes, between about 900 minutes to about 450 minutes, between about 850 minutes to about 500 minute, between about 800 minutes to about 550 minutes, between about 750 minutes to about 600 minutes or between about 700 minutes to about 650 minutes.

In some embodiments, the chamber is pie wedge shaped. In some embodiments, the chamber is regular or irregular polygon shaped. In some embodiments, the chamber is concave polygon shaped. In some embodiments, the chamber is convex polygon shaped. In some embodiments, the chamber is trigon shaped. In some embodiments, the chamber is quadrilateral polygon shaped. In some embodiments, the chamber is pentagon shaped. In some embodiments, the chamber is hexagon shaped. In some embodiments, the chamber is equilateral polygon shaped. In some embodiments, the chamber is equiangular polygon shaped. In some embodiments, the chamber is heptagon shaped. In some embodiments, the chamber is octagon shaped. In some embodiments, the chamber is nonagon shaped. In some embodiments, the chamber is decagon shaped. In some embodiments, the chamber is hendecagon shaped. In some embodiments, the chamber is dodecagon shaped. In some embodiments, the chamber is tridecagon shaped. In some embodiments, the chamber is tetradecagon shaped. In some embodiments, the chamber is pentadecagon shaped. In some embodiments, the chamber is hexadecagon shaped. In some embodiments, the chamber is heptadecagon shaped. In some embodiments, the chamber is octadecagon shaped. In some embodiments, the chamber is enneadecagon shaped. In some embodiments, the chamber is icosagon shaped. In some embodiments, the chamber is n-gon shaped. In some embodiments, an edge of the chamber is rounded. In some embodiments, an edge of the chamber comprises a baffle.
In some embodiments, the body portion of the chamber comprises at least three flat polygonal surfaces. In another embodiment, the body portion of the chamber comprises one, two, three, four, five, six, seven, or eight flat polygonal surfaces. In some embodiments, the chamber comprises six flat polygonal surfaces. In some embodiments, one or more of the polygonal surfaces are rounded.

In some embodiments, the cross-section of the body portion comprises a polygonal shape. In some embodiments, the polygonal shape comprises a trigonal, tetragonal, pentagonal, or hexagonal shape. In some embodiments, the cross-section of the body portion comprises a hexagonal shape. In some embodiments, each chamber of a plurality of chambers, comprise a hexagonal shaped cross section. In some embodiments, the plurality of chambers, each chamber comprising the hexagonal shaped cross section, comprise a hexagonally packed arrangement in a cartridge. In some embodiments, each chamber of the plurality comprising a hexagonally shaped cross-section, may be arranged in a honey-comb shaped structure in the cartridge. In some embodiments, without being bound to theory, the hexagonal shape may best fill a plane with equal size units without leaving out unused space. In some embodiments, hexagonal packing may minimize the perimeter for a given area. In some embodiments, hexagonal packing may minimize the perimeter for a given area, for example, with 120-degree angles. In some embodiments, the hexagonal shape of the chamber ensures less use of raw material.

In some embodiments, at least a portion of the chamber is circular shaped. In some embodiments, at least a portion of the cartridge is regular elliptic shaped. In some embodiments, a bottom surface of the chamber is rounded. In some embodiments, a bottom surface of the chamber is pointed.

In some embodiments, the chamber is comprised in a cartridge. In some embodiments, the cartridge comprises a plurality of chambers. In further embodiments, the chambers in the cartridge have different shapes from each other. In some embodiments, each of the chambers in the cartridge have the same or similar shapes. In some instances, the chambers in the cartridge have mixed shapes, where at least 25% are of one shape, and at least 25% are another shape, for example, 35% of the chambers in the cartridge are in the shape of a square, 25% of the chambers in the cartridge are in the shape of a wedge and the remaining 40% are in the shape of a polygon.

In some embodiments, the first chamber touches at least one side wall of the second chamber. In some embodiments, the first chamber does not touch any side wall of the second chamber. In some embodiments, the first chamber touches at least one edge of the second chamber. In some embodiments, the first chamber does not touch any edge of the second chamber.

In some embodiments, a material of the chamber comprises a material that is resistant to, e.g., cleaning procedures (chemically resistant), extreme temperatures (e.g., denaturation of nucleic acids), extreme pH values (such as those encountered during sanitization of the reactor with bases and acids, e.g. with NaOH), mechanical forces (e.g., friction caused by magnetic particles), and/or corrosion. In additional embodiments, the material of the chamber comprises a material of proper light permeation (transparent, translucent, or opaque) to a corresponding purpose. In some embodiments, the material of the chamber comprises a material of proper gas permeation to a corresponding purpose. In further embodiments, the materials of the chamber should be temperature conductive at working temperatures around 20°C (e.g., W/(mK) values of at least 10, preferably at least 15). In some embodiments, the inner surface of the chamber comprises a surface material that does not release unwanted compounds that may contaminate the end product. In further embodiments, the materials of the chamber and/or the inner surface of the chamber are PC (polycarbonate), PP (polypropylene), PAI (polyamide-imide) (e.g. Torlon), PI (polyimide) (e.g. Tecasint), PPS (polyphenylsulfide) (e.g. Tecatron), PPSU (polyphenylsulfone) (e.g. Tecason P), PSU (Polysulfone) (e.g. Tecason S), PEI (polyetherimid) (e.g. Tecapei), glass (e.g. borosilicate glass), technical ceramics (e.g. FRIDURIT^{®}), Polyaryletherketone (e.g., Polyetheretherketon (PEEK)), thermoplastics (e.g. DuraForm^{®} Pa or DuraForm^{®} GF), all of which being chemically resistant, pH resistant, and temperature resistant. In additional embodiments, the materials of the chamber comprise a material for a single-use including, but not limited to, polyethylene terephthalate and other polyethylenes, polyvinyl acetate, polyvinyl chloride. In some embodiments, the materials of the chamber comprise a material having resistance to sterilization process including steam treatment or ethylene oxide (EtO) exposure/gamma irradiation even before adding any reaction-related reagents. In some embodiments, the materials of the chamber provide protection from light (if needed) for medium contained in the chamber.

In some embodiments, the system comprises a plurality of cartridges. In some embodiments, the system comprises at least one cartridge, 2 cartridges, 3 cartridges, 4 cartridges, 5 cartridges, 6 cartridges, 7 cartridges, 8 cartridges, 9 cartridges, 10 cartridges, 11 cartridges, 12 cartridges, 13 cartridges, 14 cartridges, 15 cartridges, 16 cartridges, 17 cartridges, 18 cartridges, 19 cartridges, 20 cartridges, 21 cartridges, 22 cartridges, 23 cartridges, 24 cartridges, 25 cartridges, 26 cartridges, 27 cartridges, 28 cartridges, 29 cartridges, 30 cartridges, 31 cartridges, 32 cartridges, 33 cartridges, 34 cartridges, 35 cartridges, 36 cartridges, 37 cartridges, 38 cartridges, 39 cartridges, 40 cartridges, 41 cartridges, 42 cartridges, 43 cartridges, 44 cartridges, 45 cartridges, 46 cartridges, 47 cartridges, 48 cartridges, 49 cartridges, or 50 cartridges. A cartridge of the plurality of cartridges may comprise at least one chamber. In some embodiments, each cartridge of the plurality of cartridges comprises at least one chamber. In some embodiments, a first cartridge of the plurality of cartridges comprises the same number of chamber(s) as a second cartridge of the plurality of cartridges. In some embodiments, the first cartridge of the plurality of cartridges comprises a different number of chamber(s) as the second cartridge of the plurality of cartridges.

In some embodiments, a different reaction occurs in a single and different cartridge. In an example, in vitro transcription (IVT) occurs in one cartridge, capping of mRNA molecules in another different cartridge, and DNAase treatment in yet another different cartridge. In some embodiments, the same reactions occur in all cartridges. In some embodiments, the same reaction occurs in a plurality of cartridges. In some embodiments, a first reaction occurs in a single compartment and a second rection occurs in a plurality of cartridges. In some embodiments, n types of reactions or processes are partitioned across m chambers (which may be comprised in one cartridge or spread across a plurality of cartridges, and where n and m are positive integers and n ≤ m) such that at least one reaction or process is occurring in each chamber, but the same type of reaction or process may occur in more than one chamber, subject to the constraints of the number of reactions/processes and chambers.

In some embodiments, a subset of the plurality of chambers contains, e.g., medium or another substance but is not configured to carry out the target process. Such chambers may generally be referred to as "blanks." Any proportion of the chambers may be blanks. In some embodiments, blanks do not contain any liquid (e.g., medium). In some embodiments, blanks do not contain one or more reagents for carrying out the target process (e.g., an IVT process as described elsewhere herein).

In some embodiments, the cartridge comprises a rectangular shape. In some embodiments, the cartridge comprises a honeycomb shape. In some embodiments, the cartridge is circular shaped. In some embodiments, the cartridge is elliptic shaped. In some embodiments, the cartridge is crown shaped. In some embodiments, the cartridge comprises a plurality of rows with one or more chambers. In some embodiments, a first row of the plurality of rows has the same number of chamber(s) as a second row of the plurality of rows. In some embodiments, the first row of the plurality of rows a different number of chamber(s) as a second row of the plurality of rows.

Described herein are various embodiments of a compartment for moving a chamber and/or cartridge as described herein. Chambers and/or cartridges as disclosed herein may be comprised in one or more compartments capable of moving the chambers and/or cartridges. In some embodiments, the one or more compartments comprise a carousel. In some embodiments, the one or more compartments comprise a conveyor.

The compartment may be programmed to alter the location of the chambers and/or cartridge(s) over time. In some embodiments, the compartment is programmed to translocate a chamber and/or cartridge from a first position to a second position then back to the first position within a certain time period. The time period may be determined at least in part by a reaction or incubation time of a process occurring in the chamber/cartridge; a total number of cartridges, chambers, or compartments in the system; or a combination thereof. In some embodiments, the system is configured to carry out a first operation or set of operations at the first location and a second operation or set of operations at the second location. In an example embodiment, a reagent is added to a chamber at the first location and a synthesized compound is removed from the chamber at the second location. In this example embodiment, the compartment is programmed to translocate the chamber from the first location to the second location within a time period Y corresponding to a reaction time for synthesizing the compound from the reagent. The compartment then returns to the first location where the reagent can be added to the compartment again, allowing for a virtual "endless" cycle of production of the compound. In another example embodiment, a reagent is added to a chamber at a first location. The compartment then translocates the chamber to a second location where a second reagent is added to a chamber. The compartment then translocates the chamber to a third location where a synthesized compound is removed.

The chambers and/or cartridges may be disposed in the carousel. The carousel may rotate so as to alter the location of the chambers and/or cartridges over time. The carousel may be configured to rotate about an axis in two spatial dimensions. The carousel may be configured to rotate at a particular angular speed/velocity. The angular speed/velocity may be determined by one or more of an incubation or reaction time of a process in a chamber and/or cartridge comprised in the carousel, a desired linear speed/velocity of the chamber and/or cartridge, a dimension or other spatial parameter (e.g., length, width, height, volume) of the chamber and/or cartridge, or any combination thereof. Alternatively, or additionally, the angular velocity may be set at a predetermined rate (e.g., by an operator or by a control system as described herein).

In some embodiments, the compartments comprise a conveyor belt. The conveyor belt may be configured to move at a particular speed. The speed may be determined by one or more one or more of an incubation or reaction time of a process in a chamber and/or cartridge comprised in the carousel, a dimension or other spatial parameter (e.g., length, width, height, volume) of the chamber and/or cartridge, or any combination thereof. Alternatively, or additionally, the speed may be set at a predetermined rate (e.g., by an operator or by a control system as described herein).

Devices and systems as described herein may comprise at least one compartment. In some embodiments, the system comprises at least two compartments. In some embodiments, the system comprises at least three compartments. In some embodiments, the system comprises at least four compartments. In some embodiments, the system comprises at least five compartments. In some embodiments, the system comprises at least six compartments. In some embodiments, the system comprises at least seven compartments. In some embodiments, the system comprises at least eight compartments. In some embodiments, the system comprises at least nine compartments. In some embodiments, the system comprises at least ten compartments. In some embodiments, the compartments comprise chambers and/or cartridges that are all configured to carry out the same process (e.g., synthesis or production of compound, such as a nucleic acid compound) or subset of operations comprising a process (e.g., a purification, isolation, and/or packaging operation which is part of an overall nucleic acid compound production process) as described herein. In some embodiments, one compartment comprises chambers and/or cartridges configured to perform a synthesis of a ribonucleic acid molecule, such as by in vitro transcription (IVT), while a second compartment comprises chambers and/or cartridges configured to perform isolation of the synthesized compound. In such embodiments, the system may be configured to transport a substance (e.g., reaction mixture or potion thereof, such as a medium) from one compartment to another. The system may transport the substance by any means disclosed elsewhere herein, such as by a programmed robotic arm and/or tubing.

In some embodiments, at least one reagent is added to a chamber in a cartridge in a sequential manner. In yet other embodiments, at least one reagent is added to a portion of the total number of chambers in a cartridge in a sequential manner. In still other embodiments, at least one reagent is added to only one chamber, in two chambers, in three chambers, in four chambers, in five chambers or more in a cartridge in a sequential manner. In other embodiments, an additional amount of the same reagent is added to a chamber in a cartridge in a sequential manner. In still other embodiments, an additional amount of the same reagent is added to a portion of the total number of chambers in a cartridge in a sequential manner. In still other embodiments, an additional amount of the same reagent is added to only one chamber, in two chambers, in three chambers, in four chambers, in five chambers or more in a cartridge in a sequential manner.

In some embodiments, a medium comprises at least one reagent. In yet other embodiments, the medium comprises at least two reagents. In still other embodiments, the medium comprises three reagents. In still other embodiments, the medium comprises five or more reagents. In some embodiments, the medium with at least one reagent is loaded to a chamber before the next chamber is loaded in a sequential manner. In some embodiments, the medium with two reagents is loaded to a chamber before the next chamber is loaded in a sequential manner. In some embodiments, the medium with five or more reagents is loaded to a chamber before the next chamber is loaded in a sequential manner. In some embodiments, the reagents are pre-mixed in a separate premixing chamber and the premixed reagents are loaded to a chamber for reaction.

In some embodiments, at least one reagent is reloaded to a chamber in a cartridge after the last chamber in the cartridge is loaded with at least one reagent. In some embodiments, at least one reagent is reloaded to a chamber in a cartridge in a sequential manner after the last chamber in the cartridge is loaded with at least one reagent. In yet other embodiments, at least one reagent is reloaded to a portion of the total number of chambers in a cartridge in a sequential manner after the last chamber in the cartridge is loaded with at least one reagent. In still other embodiments, at least one reagent is reloaded in only one chamber, in two chambers, in three chambers, in four chambers, in five chambers or more in a cartridge in a sequential manner after the last chamber in the cartridge is loaded with at least one reagent. In some embodiments, at least one reagent is reloaded to a chamber in a cartridge in a sequential manner after the last chamber in the cartridge is loaded with at least one reagent, and the reagent is the same as the reagent previously loaded. In yet other embodiments, at least one reagent is reloaded to a portion of the total number of chambers in a cartridge in a sequential manner after the last chamber in the cartridge is loaded with at least one reagent, and the reagent is the same as the reagent previously loaded. In still other embodiments, at least one reagent is reloaded in only one chamber, in two chambers, in three chambers, in four chambers, in five chambers or more in a cartridge in a sequential manner after the last chamber in the cartridge is loaded with at least one reagent, and the reagent is the same as the reagent previously loaded. In some embodiments, at least one reagent is reloaded to a chamber in a cartridge in a sequential manner after the last chamber in the cartridge is loaded with at least one reagent, and the reagent is different from the reagent previously loaded. In yet other embodiments, at least one reagent is reloaded to a portion of the total number of chambers in a cartridge in a sequential manner after the last chamber in the cartridge is loaded with at least one reagent, and the reagent is different from the reagent previously loaded. In still other embodiments, at least one reagent is reloaded only one chamber, in two chambers, in three chambers, in four chambers, in five chambers or more in a cartridge in a sequential manner after the last chamber in the cartridge is loaded with at least one reagent, and the reagent is different from the reagent previously loaded.

In some embodiments, the chambers of the cartridge can be reused multiple times. In further embodiments, the chambers are rinsed/washed with at least one solution between each reaction cycle. In additional embodiments, the solution comprises deionized water, purified water, a buffered solution, a cleaning solution, or a combination thereof.

In some embodiments, a chamber in a cartridge can be drained in a sequential manner before the last chamber in the same cartridge is loaded with at least one reagent. In yet other embodiments, a portion of the total number of chambers in a cartridge can be drained in a sequential manner before the last chamber in the same cartridge is loaded with at least one reagent. In still other embodiments, only one chamber, two chambers, three chambers, four chambers, five chambers or more in a cartridge can be drained in a sequential manner before the last chamber in the same cartridge is loaded with at least one reagent.

In some embodiments, the first chamber in the cartridge can be reloaded with at least one reagent before the chamber is drained, and the at least one reagent is the same as the previously loaded reagent. In another embodiment, the first chamber in the cartridge can be reloaded with at least one reagent before the chamber is drained, and the at least one reagent is not the same as the previously loaded reagent.

In some embodiments, the contents of the chambers are agitated (e.g., stirred), so as to facilitate mixing of the contents of the chambers. In some embodiments, the contents of the chamber are mixed by vibrating or shaking the chamber. In yet other embodiments, each of the chambers are simultaneously mixed. In other embodiments, the chambers in a cartridge are mixed independently of each other. In yet other embodiments, an impeller mixes the contents of the chamber. In still other embodiments, the contents of the chamber are mixed by repeated pipetting of the contents.

In some embodiments, the chamber comprises at least one baffle, which mixes the contents of the chamber. In some embodiments, the chamber comprises an agitation device. In some embodiments, the agitation device comprises an impeller blade, a bead, a stirrer, a magnetic particle (e.g., bead), or a magnetic bar. In additional embodiments, the chamber comprises a mixer, the shaft of which can be made to rotate by a drive, as a result of which a stirring member connected rigidly to the mixer shaft is likewise made to rotate, thus mixing the substances present in the chamber. In some embodiments, the chamber comprises two or more stirring members, mostly spaced axially apart, to be arranged on and connected to the mixer shaft. The stirring member or stirring members may also be integral with the mixer shaft. In some embodiments, the mixer can be made to rotate by a magnetic drive. In further embodiment, the chamber is coupled to a control device or programmed to control in real-time a rate of agitation of the medium. The purposes of agitation or mixing include, but are not limited to, maintaining the homogeneity of the reaction mixture or medium and prevention of suspended particles or cells.

Described herein are various embodiments, of a hexagonally packed cartridge, the cartridge comprising: a plurality of hexagonal chambers, or tubes, the hexagonal chambers, or tubes configured for in vitro transcription (IVT) (e.g., to produce an mRNA molecule) and down-stream processing (DSP), and wherein cartridge is configured for each of the hexagonal chambers of the plurality to be arranged in a hexagonally-packed configuration. In some embodiments, the DSP comprises post-transcriptional capping or purification of the mRNA molecule.

Described herein are various embodiments, of a rectangularly packed cartridge, the cartridge comprising: a plurality of hexagonal chambers (e.g., chambers which comprise a hexagonal cross-section) or tubes, the hexagonal chambers or tubes configured for in vitro transcription (IVT) (e.g., to produce an mRNA molecule) and down-stream processing (DSP), and wherein the cartridge is configured for each of the hexagonal chambers of the plurality to be arranged in a rectangularly-packed configuration. In some embodiments, the DSP comprises post-transcriptional capping or purification of the mRNA molecule.

### Lid

In some embodiments, the chamber comprises a cover or lid, to prevent unwanted components from entering the chamber (for example, RNases, microbial contamination or other degrading compounds or organisms) and from shielding the content of the chamber from the outer environment. In some embodiments, the chamber comprises the lid to limit exchange with the environment. In some embodiment, the cartridge comprises a lid. In some embodiments, the lid of the cartridge is removable. In some embodiments, the lid of the cartridge is not removable.

In some embodiments, the lid can prevent excessive water evaporation and loss of other critical volatile components. In some embodiments, the lid can prevent oxidation of the reagents or any components. In some embodiments, the lid can provide with protection from light (if needed). In some embodiments, the lid prevents contamination from any other potential chemical compound.

In some embodiments, the lid of the chamber is removable. In some embodiments, the lid of the chamber is not removable. In some embodiments, the lid of the chamber comprises at least one opening for filling, draining and sampling. In some embodiments, the at least one opening is positioned on the top of the lid. In some embodiments, the lid of the chamber covers the first and possibly also the second opening.

In additional embodiments, the sample taken from the chamber through the at least one opening in the lid is transferred to, for example but not limited to, laser-based analyzers such as a flow cytometry and cell sorting devices.

In some embodiments, the cartridge is programmed to shake to mix at least one reagent. In some embodiments, the all chambers of the cartridge are configured to be shaken by an d motion (e.g., by an orbital shaker). In some embodiments, a portion of the chambers of the cartridge is configured to be shaken by an oscillating motion. In further embodiment, the cartridge is coupled to a control device or programmed to control in real-time a shaking motion.

In some embodiments, the chambers are configured to move on a conveyor belt powered by one or more powered pulleys. In some embodiments, the chamber is removable from the conveyor. In some embodiments, the chamber is not removable from the conveyor. In some embodiments, the chambers could be linked to the conveyor system using a rotation link.

In some embodiments, the chambers are configured to move within a carousel in which the chambers are disposed. In some embodiments, the chamber is removable from the carousel. In some embodiments, the chamber is not removeable from the carousel.

In some embodiments, the chamber comprises a lid. In some embodiments, the lid may be removable. In some embodiments, the lid may be configured to prevent unwanted components from entering the chamber. In some embodiments, the lid may be configured to shield the content of the chamber from the outer environment. In an embodiment, the lid may be configured to prevent evaporation of the contents of the chamber. In some embodiments, the open end of the chamber may be configured to receive the removable lid for at least partially closing the open end. In some embodiments, the lid comprises a round, square, rectangle, polygon, ellipsoid, triangle, pentagon, heptagon, octagon, nonagon, decagon, hendecagon, dodecagon, tridecagon, tetradecagon, pentadecagon, hexadecagon, heptadecagon, octadecagon, enneadecagon, icosagon, n-gon shaped, elliptic, or hexagon shape.

In some embodiments, the system comprises a chamber and a removable lid for at least partially closing the open end of the chamber. In some embodiments, the removable lid may be configured to ensure shielding the content of the chamber from the environment and also prevents evaporation.

In some embodiments, the lid comprises a push-on lid. In another embodiment, the lid comprises, for example, a screw lid, a flip top, a crown cap, a snap-on lid, a friction fit lid, a lug cap, a dome cap, a pail lid, a ribbed closure, a smooth closure, a tub lid or combinations thereof. In some embodiments, the lid is bonded to at least a portion of the chamber or a cartridge comprising the chamber. In some embodiments, the lid is adhered to at least a portion of the chamber or a cartridge comprising the chamber. In some embodiments, the lid is glued to at least a portion of the chamber or a cartridge comprising the chamber.

In some embodiments, the lid comprises an opening. In some embodiments, the lid comprises a central opening. In some embodiments, the opening may be configured for access to a handling mechanism, the handling mechanism configured to add or remove components and/or liquids in one or more chambers.

In some embodiments, the lid comprises a puncturable membrane. In some embodiments, the puncturable membrane is an elastic membrane. In some embodiments, the assembly comprises a valve or inlet.

### Identification (ID) mechanism

In some embodiments, the chamber may comprise an identification (ID) mechanism. In some embodiments, the ID mechanism may comprise an RFID tag, a smart label, a reader for reading an RFID tag or smart label, or a combination thereof. In some embodiments, the Smart labels may comprise QR codes, bar codes, or a combination thereof. In some embodiments, processing steps of the chamber may be determined by the ID of the chamber. In some embodiments, the ID of the chamber may be transmitted to a processor, which based on this information determines the processing step of the chambers and transmits a signal to a processing mechanism to execute the step. In an embodiment, the cartridge may comprise one or more ID mechanisms. In some embodiments, the cartridge may comprise an RFID tag.

### Aperture

In some embodiments, the cartridge comprises at least one aperture. In some embodiments, the aperture is configured to engage with a gripping mechanism. In some embodiments, a robotic arm or handling device comprises the gripping mechanism. In some embodiments, the cartridge comprises at least two apertures. In some embodiments, the apertures are positioned at the upper surface of the cartridge.

### Robotic Arm and/or Handling Device

In some embodiments described herein, the robotic arm is programmed to operate by the at least one computer processor. In some embodiments, the robotic arm is a static arm under the control of at least one computer processor. In some embodiments, the robotic arm is movable and under the control of at least one computer processor. In still other embodiments, the robotic arm is capable of moving in at least an X-axis of direction relative to the cartridge. In other embodiments, the robotic arm is capable of moving in at least a Y-axis of direction relative to the cartridge. In other embodiments, the robotic arm is capable of moving in at least a Z-axis of direction relative to the cartridge. In one embodiment, the robotic arm is capable of moving in both an X-axis and a Y-axis relative to the cartridge. In other embodiments, the robotic arm is capable of moving in at least two directions consisting of an X-axis, Y-axis and Z-axis relative to the cartridge.

In some embodiments, the system comprises at least one robotic arm. In some embodiments, the system comprises one robotic arm. In some embodiments, the system comprises two robotic arms. In some embodiments, the system comprises three robotic arms. In some embodiments, the system comprises four robotic arms. In some embodiments, the system comprises five robotic arm. In some embodiments, the system comprises six robotic arms. In some embodiments, the system comprises seven robotic arms. In some embodiments, the system comprises eight robotic arms. In some embodiments, the system comprises nine robotic arms. In some embodiments, the system comprises ten robotic arms. In some embodiments, the system comprises eleven robotic arms. In some embodiments, the system comprises twelve robotic arms.

In some embodiments, the robotic arm is human finger shaped. In some embodiments, the robotic arm is human hand shaped. In some embodiments, the robotic arm is human arm shaped. In some embodiments, the robotic arm is one arm. In some embodiments, the robotic arm is one hand. In some embodiments, the robotic arm is five fingers. In some embodiments, the robotic arm is two arms. In some embodiments, the robotic arm is two hands. In some embodiments, the robotic arm is ten fingers.

In some embodiments, the robotic arm is positioned in the vicinity of the cartridge in a manner that each chamber can be reached by the robot and the robot arm is covering all the chambers. In some embodiments, the chambers are movable and may be moved closer to the robot arm to dispense or remove medium and/or reagent from a chamber (e.g., by a carousel or conveyor belt). In some embodiments, a plurality of chambers is positioned to surround a robotic arm. In some embodiment, the robotic arm is located above the chambers or can be installed next to the cartridge.

In some embodiments, the at least one robotic arm is removable from the system. In another embodiments, the at least one robotic arm is not removable from the system.

In some embodiments, the at least one robotic arm loads the at least one reagent to the chambers. In some embodiments, the at least one robotic arm removes the compound from the chamber.

In some embodiments, the system comprises one or more robotic arms. In some instances, such as when a plurality of robotic arms is present, different reagents can be provided by different robots. In some instances, when more than one robotic arm is present, evacuation of the chamber content can be performed by a separate robotic arm. In some embodiments, the evacuation of the content of the chamber is performed by the same robotic arm. In some embodiments, the number of robotic arms is determined at least in part based on a time interval between filling a first chamber and a second chamber (sometimes referred to herein as a time interval X), a time interval between adding a medium or portion thereof to a chamber and removing a synthesized compound from the chamber (sometimes referred to herein as a time interval Y), a number of chambers, a number of cartridges, or any combination thereof. In an example embodiment, a robotic arm is programmed to sequentially add to a plurality of chambers a reagent which participates in the synthesis of a compound. The robotic arm is further programmed to remove the synthesized compound from the plurality of chambers after a time interval Y, where Y is a reaction or incubation time for producing a compound with the added reagent. The synthesized compound may be comprised in a medium which is removed from the chamber by the robotic arm. In another example embodiment, a robotic arm is programmed to sequentially add to a plurality of chambers a reagent which participates in the synthesis of a compound. A second robotic arm is programmed to remove the synthesized compound from the plurality of chambers after a time interval Y, where Y is a reaction or incubation time for producing a compound with the added reagent. The synthesized compound may be comprised in a medium which is removed from the chamber by the second robotic arm.

In some embodiments, the at least one robotic arm is equipped with pipettes, micropipettes, needles or tips for delivering the reagents to the chambers. In some embodiment, the pipettes, micropipettes, needles or tips is removable and/or single-use. In some embodiment, the pipettes, micropipettes, needles or tips is not removable.

In some embodiments, the at least one robotic arm is programmed to transfer the at least one reagent to and from the chambers.

In some embodiments, the at least one robotic arm is programmed to add the reagents to the chamber during the reaction, if necessary.

In some embodiments, the programming of the at least one robotic arm is based at least in part on the number of chamber and/or cartridges, a time interval between adding or removing a substance form subsequent chambers, a reaction or incubation time, an overall processing time, or any combination thereof.

### Separation and/or Purification Device

The present disclosure provides various embodiments of a device for separating and/or purifying a compound, the compound in accordance with some embodiments. In some embodiments, the device for separating and/or purifying a compound comprise a chamber, a cartridge, a lid, a sensor, or any combination thereof, as described elsewhere herein with respect to reactor systems for, e.g., production and/or synthesis of the compound. In some embodiments, the device for separating and/or purifying the compound is the same as the device for producing and/or synthesizing the compound or is comprised in a part of the device or system for producing and/or synthesizing the compound. In some embodiments, the device for separating and/or purifying the compound is separate from the device for producing and/or synthesizing the compound. In such embodiments, the compound is transferred from the synthesis device to the purification/separation device by manual intervention (e.g., of a human operator) or automatically, such as by a robotic arm or other handling device as described elsewhere herein.

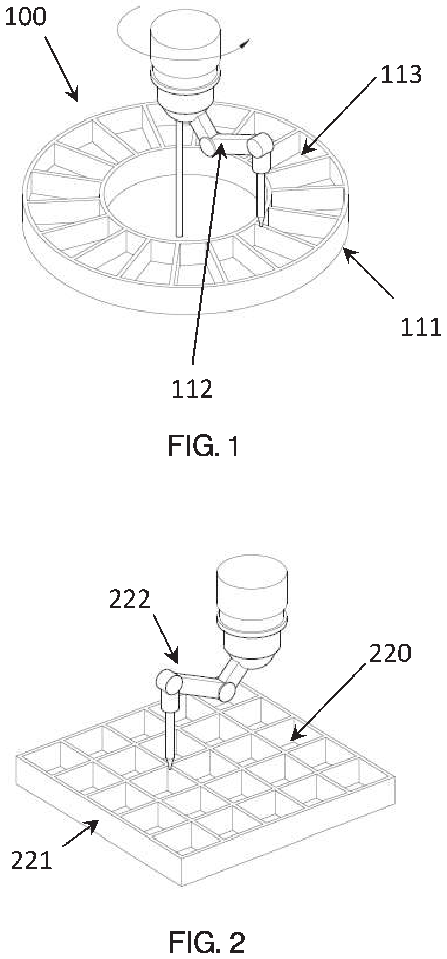

In some embodiments, the device for separating and/or purifying a compound comprises a carousel. **FIG. 1** depicts a non-limiting example a carousel 100. In some embodiments, the device for separating and/or purifying a compound may be automated. In some embodiments, the device for separating and/or purifying a compound may comprise a sample plate. In some embodiments, the sample plate may comprise sample holders. In some embodiments, the sample holders are configured to hold sample containers comprising the compound in a liquid medium in a certain position. In some embodiments, a magnet unit may be positioned at each sample holder. In some embodiments, magnetic particles may be introduced into the samples and mixed with the compound. In some embodiments, magnetic particle-bound compound may be subjected to washing and elution steps, inside the device. In some embodiments, the compound is referred to as the compound of interest. In the same or another embodiment, the carousel 100 depicted in **FIG. 1** is used for production of the compound.

In one aspect, robotic arms, injectors, and/or pumps are used for dispensing and removing components and liquids in the sample containers positioned within the device for separating and/or purifying a compound.

In some embodiments, the device for separating and/or purifying a compound comprises: a sample plate, the sample plate comprising a base portion and one or more sample holders provided on the base portion for receiving a sample container, the sample container configured to hold a liquid sample comprising the compound and magnetic particles; and a magnet unit positioned at each sample holder, the magnet unit configured to capture the magnetic particles or introduce a movement of the magnetic particles, and wherein the sample holders are configured to perform a mechanical motion, such that the magnetic particles are mixed with the liquid.

### System Controller (Computer Processor)

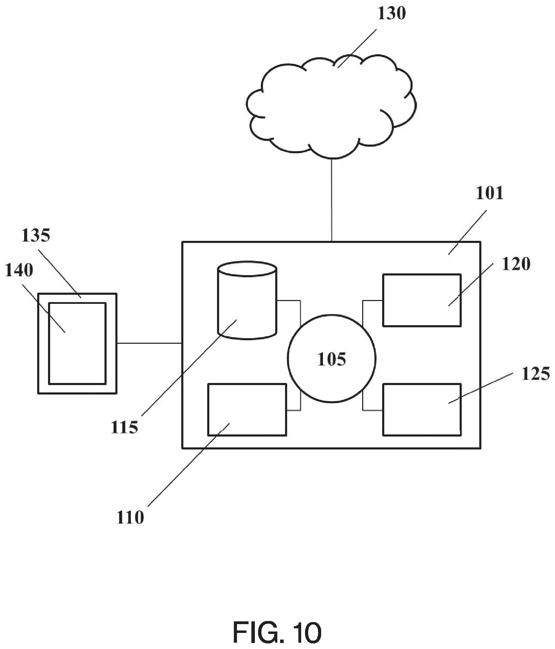

The present disclosure provides computer control systems that are programmed to implement methods of the disclosure. **FIG. 10** shows a computer system 101 that is programmed or otherwise configured to control at least one robotic arm and the system. The computer system 101 can regulate various aspects of the present disclosure, such as, for example, performance of at least one robotic arm. The computer system 101 can be an electronic device of a user or a computer system that is remotely located with respect to the electronic device. The electronic device can be a mobile electronic device.

The computer system 101 includes a central processing unit (CPU, also "processor" and "computer processor" herein) 105, which can be a single core or multi core processor, or a plurality of processors for parallel processing. The computer system 101 also includes memory or memory location 110 (e.g., random-access memory, read-only memory, flash memory), electronic storage unit 115 (e.g., hard disk), communication interface 120 (e.g., network adapter) for communicating with one or more other systems, and peripheral devices 125, such as cache, other memory, data storage and/or electronic display adapters. The memory 110, storage unit 115, interface 120 and peripheral devices 125 are in communication with the CPU 105 through a communication bus (solid lines), such as a motherboard. The storage unit 115 can be a data storage unit (or data repository) for storing data. The computer system 101 can be operatively coupled to a computer network ("network") 130 with the aid of the communication interface 120. The network 130 can be the Internet, an internet and/or extranet, or an intranet and/or extranet that is in communication with the Internet. The network 130 in some cases is a telecommunication and/or data network. The network 130 can include one or more computer servers, which can enable distributed computing, such as cloud computing. The network 130, in some cases with the aid of the computer system 101, can implement a peer-to-peer network, which may enable devices coupled to the computer system 101 to behave as a client or a server.

The CPU 105 can execute a sequence of machine-readable instructions, which can be embodied in a program or software. The instructions may be stored in a memory location, such as the memory 110. The instructions can be directed to the CPU 105, which can subsequently program or otherwise configure the CPU 105 to implement methods of the present disclosure. Examples of operations performed by the CPU 105 can include fetch, decode, execute, and writeback.

The CPU 105 can be part of a circuit, such as an integrated circuit. One or more other components of the system 101 can be included in the circuit. In some cases, the circuit is an application specific integrated circuit (ASIC).

The storage unit 115 can store files, such as drivers, libraries and saved programs. The storage unit 115 can store user data, e.g., user preferences and user programs. The computer system 101 in some cases can include one or more additional data storage units that are external to the computer system 101, such as located on a remote server that is in communication with the computer system 101 through an intranet or the Internet.

The computer system 101 can communicate with one or more remote computer systems through the network 130. For instance, the computer system 1101 can communicate with a remote computer system of a user. Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 101 via the network 130.

Methods as described herein can be implemented by way of machine (e.g., computer processor) executable code stored on an electronic storage location of the computer system 101, such as, for example, on the memory 110 or electronic storage unit 115. The machine executable or machine-readable code can be provided in the form of software. During use, the code can be executed by the processor 105. In some cases, the code can be retrieved from the storage unit 115 and stored on the memory 110 for ready access by the processor 105. In some situations, the electronic storage unit 115 can be precluded, and machine-executable instructions are stored on memory 110.

The code can be pre-compiled and configured for use with a machine having a processer adapted to execute the code or can be compiled during runtime. The code can be supplied in a programming language that can be selected to enable the code to execute in a pre-compiled or as-compiled fashion.

Aspects of the systems and methods provided herein, such as the computer system 101, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 101 can include or be in communication with an electronic display 135 that comprises a user interface (UI) 140 for providing. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 105.

### Sensors

In some embodiments described herein, the system comprises one or more sensors or probes for monitoring one or more operational parameters in real-time including, but not limited to, a liquid level sensor, a thermometer, a pH probe, an oxygen probe, a carbon dioxide probe, a rate of agitation sensor, combinations thereof, and the like. These sensors can detect one or more operational parameters and/or monitor physical properties and chemical compositions, combinations thereof, and the like of the medium or the chamber headspace.

In some embodiments, the chamber is configured to detect a contamination of the at least one reagent. In some embodiments, the chamber is configured to detect a contamination of more than one reagent.

In some embodiments, the chamber is configured to monitor and/or regulate a pH value of a medium comprising the at least one reagent. In further embodiments, the chamber comprises a pH probe to monitor a pH value of a medium real-time.

In some embodiments, the chamber is configured to monitor and/or regulate a temperature of a medium comprising the at least one reagent or an atmosphere with the chamber. In further embodiments, the chamber comprises a thermometer. In additional embodiments, the chamber comprises a thermal probe to monitor a temperature of a medium in real-time.

In some embodiments, the chamber is configured to monitor and/or regulate a salt concentration of a medium comprising the at least one reagent.

In some embodiments, the chamber is configured to monitor and/or regulate osmolarity of a medium comprising the at least one reagent. In some embodiments, the chamber comprises a device for measuring osmolarity of a medium in real-time.

In some embodiments, the chamber is configured to monitor and/or regulate conductivity of a medium comprising the at least one reagent. In some embodiments, the chamber comprises an electrical conductivity meter to monitor conductivity of a medium in real-time.

In some embodiments, the chamber is configured to monitor and/or regulate turbidity of the medium comprising the at least one reagent. In further embodiments, the chamber comprises a turbidity tube to monitor turbidity in real-time.

In some embodiments, the chamber is configured to monitor and/or regulate a rate of agitation of the medium comprising the at least one reagent. In some embodiments, the chamber comprises an agitation sensor to monitor a rate of agitation of the medium in real-time.

In some embodiments, the chamber is configured to monitor and/or regulate volume of the medium comprising the at least one reagent. In some embodiments, the chamber comprises a liquid lever sensor to monitor volume of the medium in real-time.

In some embodiments, the chamber is configured to monitor and/or regulate humidity of the atmosphere within the chamber. In additional embodiments, the chamber comprises a hygrometer to monitor humidity of the atmosphere within the chamber.

In some embodiments, the chamber is configured to monitor and/or regulate O₂ concentration of the atmosphere within the chamber. In some embodiments, the chamber comprises an oxygen probe to monitor oxygen concentration of the atmosphere in real-time.

In some embodiments, the chamber is configured to monitor and/or regulate CO₂ concentration of the atmosphere within the chamber. In some embodiments, the chamber comprises a carbon dioxide probe to monitor CO₂ concentration of the atmosphere within the chamber in real-time.

In some embodiments, the chamber is configured to monitor and/or regulate gas composition of the atmosphere within the chamber. In some embodiments, the chamber is configured to monitor and/or regulate gas composition of dissolved gases in the medium. In some embodiments, the dissolved gases comprise oxygen, carbon dioxide, or both.

In some embodiments, the chamber is configured to monitor and/or regulate a concentration of a molecule comprised in the medium utilizing a spectroscopic device. In some embodiments, the spectroscopy device comprises Infrared spectrometer, Raman spectrometer or UV spectrometer.

In some embodiments, the chamber is configured to monitor and/or regulate a viable cell density based on a cell concentration measurement. In some embodiments, the chamber comprises a capacitance probe to monitor the cell concentration within the chamber.

In some instances, if necessary, an aliquot of the content in the chamber is taken/transferred from the chamber during the reaction so that physical properties and/or chemical compositions of the content can be monitored in real-time. In some embodiments, the aliquot of the content in the chamber is taken/transferred by at least one robotic arm. In further embodiments, the chambers are connected to an autosampler that takes samples and routes them to any intermediate sample processing machines and/or automated analyzers.

In some embodiments, the chamber comprises an open end configured to receive at least one reagent, wherein at least part of the body portion comprises a flat polygonal surface configured to allow positioning and/or supporting a sensor in the chamber. In some embodiments, the chamber comprises a sensor and/or a probe. In some embodiments, sensors and/or probes contribute to the monitoring of processes that take place within the chamber. In some embodiments, the sensor comprises: a liquid level sensor; a rate of agitation sensor; a sensor or probe that measures the pH, the temperature, the pressure, the flow velocity, or the oxygen, carbon dioxide, magnesium, or salt levels of the components contained in the chamber; or a combination thereof. In some embodiments, the sensor may comprise a longitudinal insert, located inside the chamber.

In some embodiments, the chamber is configured for single use. In some embodiments, the chamber is configured for multiple uses.

### Additional Components

In some embodiments disclosed herein, a chamber, a storage vessel, and a robotic arm are configured respectively to transmit data.

In some embodiments, the system comprises at least one intermediate storage vessel. In some embodiments, the intermediate storage vessel is configured to store the compound. In some embodiments, the intermediate storage vessel is not in fluid communication with the chamber. In some embodiments, the storage vessel is configured to detect a contamination of the compound.

In some embodiments, the intermediate storage vessel is configured to being coupled to one or more sensors or probes for monitoring one or more physical properties and chemical compositions, combinations thereof, and the like of the compound. In some embodiments, the intermediate storage vessel contains means to perform certain chemical, physical or mechanical treatment of the compound and/or the undesired residuals - for example, binding of the product, partial elimination of residuals, or adjustment of pH value.

In some embodiments, the system comprises a purification system. In further embodiments, the purification system comprises an affinity purification system, an ion exchange system, a selective precipitation system, a chromatography system, a tangential flow filtration device or a dead end filtration device.

In some embodiments, the system comprises separate units for the delivery of reagents and the evacuation of waste, as well as for the collection of the biochemical compound. Each unit is thus designed to allow the provision of filtered, sterile air to be circulated within the units. Air filtering means comprises a HVAC system with HEPA filters. In some embodiments, the system comprises a HVAC system comprising a series of HEPA filters to protect the compound within the chamber.

In some embodiments, the compound is removed from the chamber by a pressurized system. In some embodiments, the pressurized system comprises a negative pressure. In some embodiments, the pressurized system comprises a pump configured to remove a medium comprising the compound. In some embodiments, the pump is in fluid communication with a chamber. In some embodiments, the pump is not in fluid communication with a chamber. In additional embodiments, the pump comprises a vacuum pump, a peristaltic pump, a centrifugal pump or combination thereof. In some embodiments, the compound is removed from the chamber by at least one robotic arm which is equipped with pipettes, micropipettes, needles or tips.

In some embodiments, the system comprises a modular unit. In some embodiments, the modular unit comprises a docking station configured to receive a receiving container, a cartridge or a conveyor.

In some embodiments, the modular unit comprises at least one cartridge. In some embodiments, the modular unit is movable. In some embodiments, the modular unit is stackable. In some embodiments, the modular unit comprises a plurality of receiving containers. In some embodiments, the receiving container is configured to contain at least one reagent.

In some embodiments, different modular units are assembled or constructed on site rapidly, and potentially disassembled with similar rapidity. In some embodiments, if necessary, the modular units and/or the cartridges can be added for a particular need or process setting. In some embodiment, the modular unit is configured to be coupled to a heat transfer device. In some embodiments, the modular unit comprises an agitation device. In some embodiments, the modular unit comprises one or more robotic arms. In some embodiments, the modular unit comprises one or more pumps. In some embodiments, the modular unit comprises a purification system. In some embodiments, the modular unit comprises an air filtration system. In some embodiments, the modular unit comprises a controller system.

In further embodiments, the two separate cartridges can accommodate two separate steps of the reaction so that the first step of the reaction occurs in a first cartridge and the second step of the reaction occurs in a second cartridge. In some embodiments, the reaction conditions and/or reaction parameters of two separate steps of the reaction are separately controlled by the computer processor.

In some embodiments, the plurality of chambers is disposed on the conveyor. In some embodiments, the modular unit comprises at least one conveyor. In some embodiments, at least one conveyor is stackable.

In some embodiments, the system comprises a waste collecting vessel, which facilitates draining and replacement in a controlled manner. In some embodiments, the waste collecting vessel may be connected to the system with a valve that is turned on to remove waste from the system when desired.

### Methods

Provided herein, in one embodiment, is a method of producing a compound, comprising: (a) providing a plurality of chambers, and a chamber of the plurality of chambers comprises a medium comprising at least one reagent; and (b) using at least one computer processor to generate instructions to: (i) fill a chamber with the medium; (ii) repeat (i) until at least a portion of the plurality of chambers is filled with the medium; (iii) synthesize the compound in a chamber that is filled with the medium and remove the compound from the chamber that is filled with the medium after a time interval Y; (iv) rinse/wash the chamber and (v) continuously re-load, synthesize, and remove the compound.

In some embodiments, the method comprises removing the compound from the chamber. In some embodiments, the method comprises removing the medium through a filter so that the compound is retained in the chamber.

In some embodiments of the present disclosure, the method comprises providing a robotic arm coupled to the at least one computer processor and configured to receive the instructions in (b).

In some embodiments, the method comprises filling a subsequent chamber after a time interval X. In some embodiments, the time interval Y is at least the time interval X times the number of the plurality of chambers.

In some embodiments are described methods comprising the at least one computer processor to generate instructions to (1) fill a chamber with the medium and remove the medium comprising the compound, (2) rinse the chamber to avoid carry-over between reactions and (3) repeat (1)-(2) in the same chamber until receiving an input for stop.

In some embodiments, the first reagent is different from the second reagent. In some embodiments, the first reagent is the same as the second reagent.

In some embodiments, the first chamber and the second chamber comprise a substantially same temperature. In another embodiments, the first chamber and the second chamber comprise different temperatures.

In some embodiments, the first chamber and the second chamber comprise a substantially same reaction time. In some embodiments, the first chamber and the second chamber comprise different reaction times.

In additional embodiments, the at least one robotic arm is programed to load the reagents to the same chamber more than once, and the reagents are all different from each other, before loading the reagent to the next chamber. In some embodiments, the at least one robotic arm is programed to load the medium with at least one reagent to a chamber before the next chamber is loaded in a sequential manner. In some embodiments, the at least one robotic arm is programed to load the medium with two reagents to a chamber before the next chamber is loaded in a sequential manner. In some embodiments, the at least one robotic arm is programed to load the medium with five or more reagents to a chamber before the next chamber is loaded in a sequential manner.

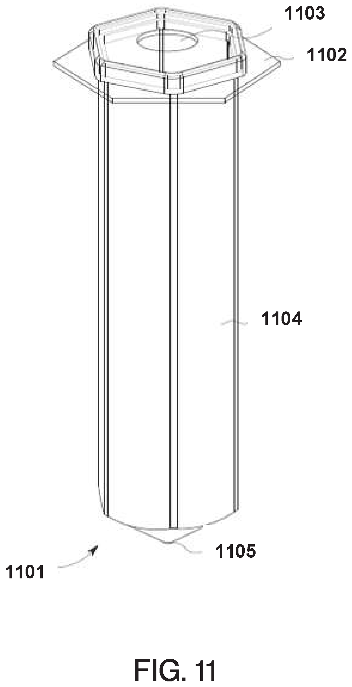

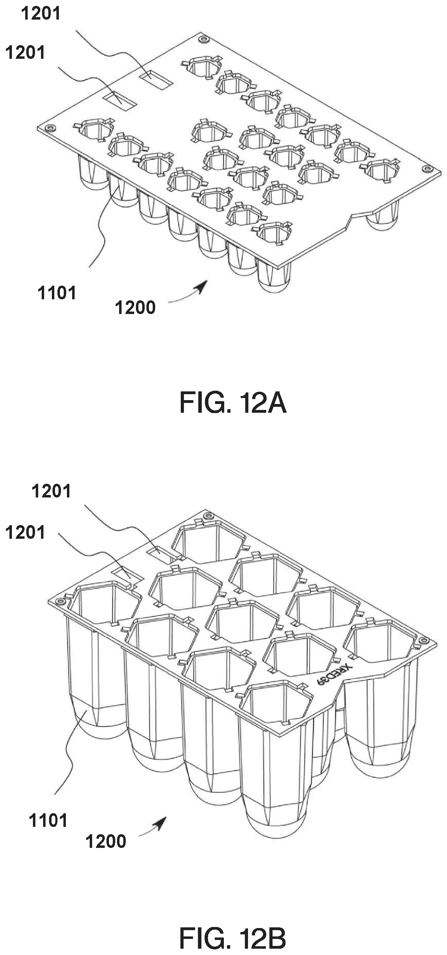

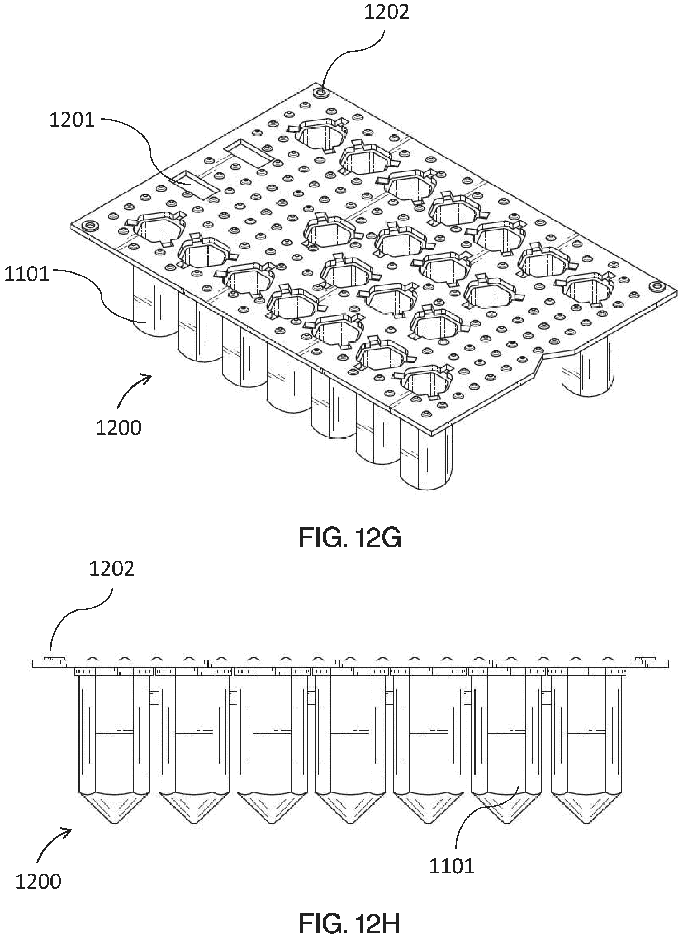

Described herein are various methods of use of chambers 1101 as depicted in **FIG. 11****.** In some embodiments, the method may comprise the use of a plurality of chambers 1101, wherein at least one chamber of the plurality of chambers 1101 may remain empty. In some embodiments, the method of use of a chamber 1101 comprises a production process. In some embodiments, the method of use comprises transporting the cartridge 1200 (as depicted in, e.g., **FIG. 12A**) to a location of a production system. In some embodiments, the method comprises transferring the cartridge from a first location to a second location.

Described herein are various methods utilizing the systems and devices described elsewhere herein in accordance with some embodiments. In some embodiments, the method may comprise producing and/or synthesizing a nucleic acid. In some embodiments, methods may comprise producing and/or synthesizing RNA. In some embodiments, methods may comprise producing and processing biomolecules such as DNA, RNA, and protein. In some embodiments, the method may comprise in vitro transcription of RNA. In some embodiments, the method may comprise mixing IVT reagents in a premix. In some embodiments, methods utilizing the chambers and/or cartridges may perform the IVT reaction. In some aspects, chambers or cartridges in accordance with some embodiments, may be used for downstream processing of RNA. In an embodiment the chambers and cartridges may be used for post-transcriptional capping of RNA. In another embodiment the chambers and cartridges may be used for co-transcriptional capping of RNA.

Alternatively, the chambers or cartridges as disclosed herein may be used for DNA synthesis. In some embodiments, the chambers or cartridges may be used for downstream processing of DNA after synthesis. In some embodiments, the method may comprise a DNA synthesis method comprising thermocycling. In some embodiments, chambers or cartridges in conjunction with the DNA-preparation method for plasmid amplification may be used, for example.

Described herein are various embodiments of a method for separating and/or purifying a compound, using devices and the systems disclosed herein. In some embodiments, the method comprises: (a) providing one or more sample containers comprising a compound and magnetic particles in a liquid medium, wherein each sample container is positioned in a sample holder; (b) allowing the compound to mix with the magnetic particles by mechanical motion of the sample holder; (c) capturing or introducing a movement of the magnetic particles towards a magnet unit present in the vicinity of the sample holder, thereby causing a separation of the liquid and the magnetic particles; and (d) removing the liquid. In some embodiments, the method further comprises adding a liquid to the magnetic beads. In some embodiments, the method further comprises repeating (b) to (d). In a further embodiment, (b) to (d) are repeated at least 2 to 10 times, 2 to 8 times, 2 to 7 times, 2 to 6 times, 2 to 5 times, 2 to 4 times, or 2 to 3 times, such as at least 2 to 5 times. Alternatively, (b) to (d) are repeated at least 3 to 10 times, 4 to 10 times, 5 to 10 times, 6 to 10 times, 7 to 10 times, 8 to 10 times, or 9 to 10 times.

Described herein are various embodiments of a method comprising: providing or obtaining the chamber as described in any one of the preceding claims; and producing a nucleic acid. The method of any one of the preceding claims, wherein the nucleic acid is a ribonucleic acid (RNA).

### Example Reactor Systems

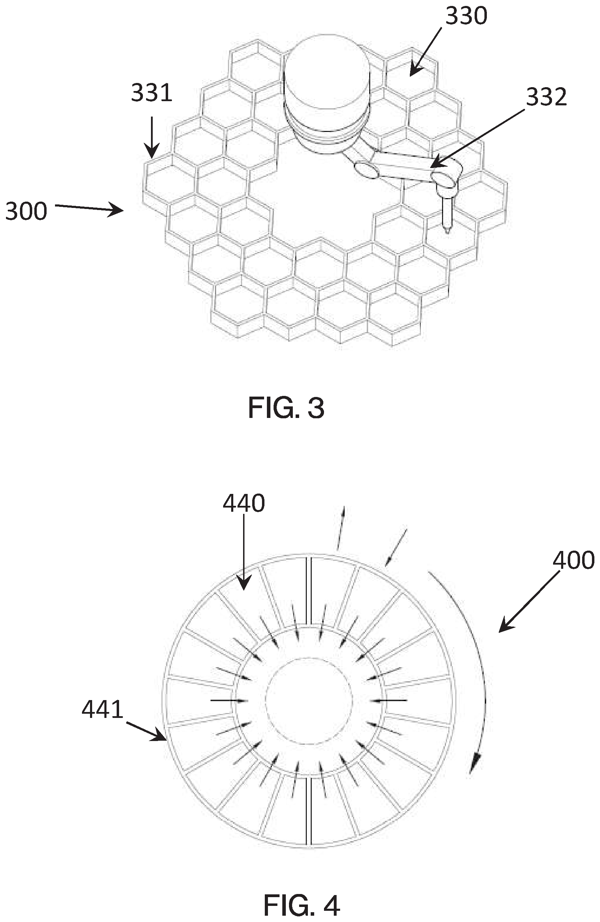

In some embodiments, as shown in **FIG. 1****,** the chambers 113 are arranged in a circular manner, divided from each other by upstanding walls. In some embodiments, as illustrated in **FIGS. 2** and **3****,** the chambers are respectively arranged in a rectangular manner or in a honeycomb structure.

In some embodiments, the chambers comprise an extended body with a particular cross-sectional geometry, such as those depicted in **FIGS. 11** and **12A****-12J.**

In further embodiments, the chambers may be comprised in cartridges, such as those depicted in **FIGS. 12A-12J****.**

In some embodiments, as shown in **FIGS. 1** and **2****,** the chambers are not in fluid connection with each other. The chambers are thus designed to allow retrieving and holding multiple reagents by means of a robotic arm located in the vicinity of said cartridge and chambers. In **FIG. 1****,** the robotic arm 112 is located in a middle opening of the cartridge. In some embodiments, as shown in **FIG. 2****,** the robotic arm 222 is located above the chambers 220 disposed in the cartridge 221, or can be installed next to the cartridge (not shown). It will be clear that other arrangements not depicted in the Figures equally fall within the scope of the current invention.

Each chamber is designed to receive at least one reagent or multiple reagents via a first opening. In some embodiments, a second opening may be present, different from the first opening, for the evacuation of the content of said chambers. In some embodiments, the chamber content may be evacuated by opening or retracting fully or partially the bottom of the chamber, causing the chamber content to flow downwards, for instance to a reservoir or a channel. In another embodiment, each chamber may be equipped with channels or tubing allowing the evacuation of the chamber content. The chambers are thus designed to behave as small reactor vessels, for the production of biochemical molecules. The volume of each chamber is preferably between 1 and 50 ml, although the dimensions can be adapted depending on the intended use.

The chambers may be provided by a cover or lid, to prevent unwanted components from entering said chamber (e.g., RNases) and from shielding the content of said chamber from the outer environment. Said lid or cover may cover the first and possibly also the second opening. In an embodiment, the cover or lid is a pierceable membrane or provided with a valve or inlet for allowing the entrance of reagents and/or the robotic arm.

In an alternate embodiment, two or more chambers of the cartridge may be in fluid connection with each other. For example, the chambers 330 positioned in the outer ring of the cartridge 331 of **FIG. 3** may be provided with an opening to allow fluid to flow into the corresponding chambers in the inner ring. An appropriate means to transfer the fluid from one chamber to the corresponding chamber, could be to tilt the chambers in the outer ring, causing the liquid to flow in the corresponding chambers in the inner ring. This evacuation must be conducted before the robotic arm resupplies the chambers in the outer ring with reagents. The honeycomb structure has as an advantage that it is more compact than, for instance, the embodiment shown in **FIG. 1****.**

The robotic arm may be equipped with (micro)pipettes or tips for delivering the reagents to the chambers. In an embodiment, the pipettes or tips may be removable and/or single-use. Required reagents can be delivered by the robotic arm to the chambers. To that purpose, in an embodiment the head of the robotic arm is movable from a reagent reservoir to the opening of the chambers. Process conditions in these chambers, such as temperature, pH, reaction speed, etc. can be closely monitored by a process controller (not shown).

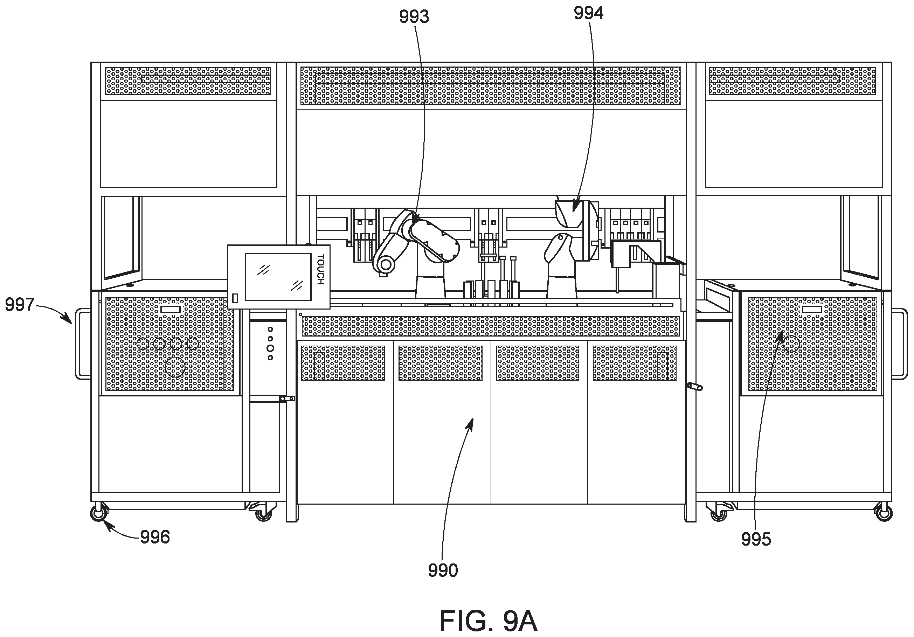

This set-up allows for the design of the overall system which requires fewer upscaling steps and guarantees a high quality biomolecule product due to the strict process control. Furthermore, the small size of the system facilitates operation in an isolator, a biosafety cabinet, or other compartment, such the one as shown in **FIGS. 9A-9B****.**

In another embodiment, as shown in **FIG. 1****,** the chambers 113 comprise a single opening through which the reagents are administered, and the products are evacuated. These operations are conducted by the robotic arm 112 in the middle of the carousel 100. The robotic arm 112 rotates from one chamber 113 to the next, each time first evacuating the content of the chamber, rinsing/washing the chamber and subsequently administering the reagents again, thereby, assuring a semi-continuous product flow.

**FIG. 2** illustrates another embodiment of a cartridge 221 wherein the chambers 220 are positioned in a rectangular manner, divided from each other by upstanding walls. The robotic arm 222 is positioned directly above the chambers but could also be positioned in the vicinity of the cartridge. As for the embodiment of **FIG. 1****,** the robotic arm 112 serves to add reagents to openings of the chamber 113. Evacuation of the content of the chambers 113 may be performed by the same robotic arm 112, or a second robotic arm (not shown). In another embodiment, evacuation may occur via a second opening, configured to evacuate the content of one or more chamber. Said second opening could be thus positioned that it allows evacuation from underneath said chamber.

**FIG. 3** further illustrates yet another embodiment of a cartridge 331 wherein a cartridge 331 comprises chambers 330 in a honeycomb structure. Again, a robotic arm 332 is positioned in the vicinity of the cartridge 331 such as in an opening in the middle of cartridge 331, allowing manipulation of said chambers 330 (adding of reagents and/or evacuating content).

In an embodiment, the chambers 330 as shown in **FIG. 3** are not in fluid connection with each other. However, in an alternate embodiment, two or more chamber of the cartridge may be in fluid connection with each other. For example, the chambers 330 positioned in the outer ring of the cartridge 331 of **FIG. 3** may be provided with an opening to allow fluid to flow into the corresponding chambers in the inner ring. An appropriate means to transfer the fluid from one chamber to the corresponding chamber, could be to tilt the chambers in the outer ring, causing the liquid to flow in the corresponding chambers in the inner ring. This evacuation must be conducted before the robotic arm resupplies the chambers in the outer ring with reagents. The honeycomb structure has as an advantage that it is more compact than, for instance, the embodiment shown in **FIG. 1****.**

**FIGS. 1** to **4** show only one robotic arm but it will be appreciated by those skilled in the art that multiple robotic arms can be present, for instance when different reagents are to be provided by different robots or when evacuation of the chamber content is performed by a separate robotic arm.

In one embodiment, the cartridge may be static. In another embodiment, as depicted in **FIG. 4****,** the cartridge 441 may be allowed to perform a movement, such as a rotation (see arrows in **FIG. 4****,** indicating the rotation of the cartridge). This embodiment allows for a more static robotic arm since the rotational movement of the cartridge 441 brings those chambers 440 that require the addition of a reagent or evacuation in the vicinity of the robotic arm, rather than the robotic arm moving to each of the chambers. The system may thus be programmed such that the cartridge is rotated in a predefined manner, such as with a particular rotational speed (e.g., determined based at least in part on a reaction/incubation time or number of chambers). As for the embodiments shown in **FIGS. 1** to **3****,** the robotic arm may provide reagents to the opening of the chambers and may be configured to evacuate the content of the chambers. Alternatively, evacuation may occur by means of the second opening of the chamber. This second opening may be present at the bottom of each chamber. The second opening could be positioned on the top of the covering lid, with the system to suck the liquid, like a dip tube for example.

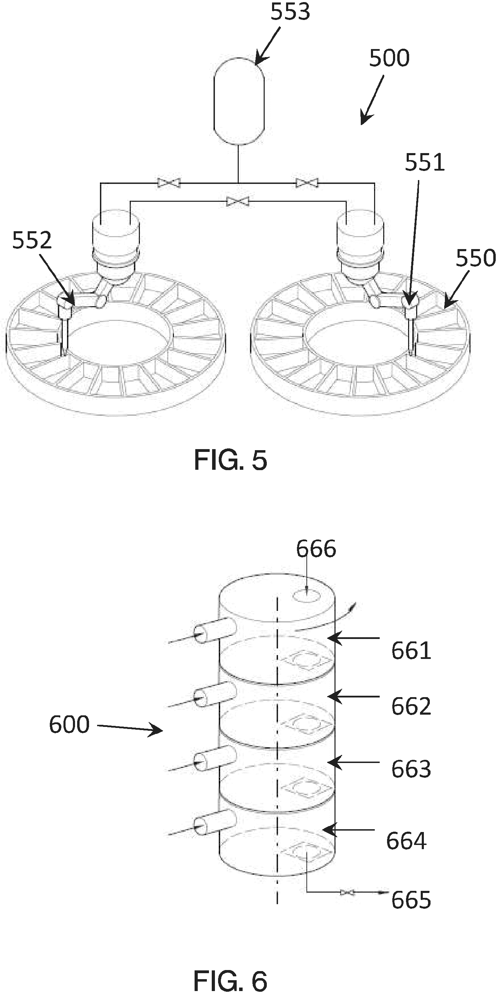

A particular embodiment is shown in **FIG. 5****,** wherein the plurality of cartridges 550 and robotic arms 551 & 552 are provided. The embodiment of **FIG. 5** allows several cartridges 550 to be worked on in parallel, which may enhance the output and the efficiency of the production of biochemical molecules. Alternatively, the configuration shown in **FIG. 5****,** or variants thereof, may be used to perform a sub-reaction in a first cartridge, after which the content of the first cartridge is transferred to a second cartridge for further reaction. Meanwhile, the first cartridge and its chambers may be provided with reagents to start a new sub-reaction. Again, the design in **FIG. 5** may enhance the final output of the system.

In some embodiments, transfer of content from a first to a subsequent cartridge and/or corresponding subsequent chamber may be performed by the robotic arm. In another instance, the embodiment as shown in **FIG. 5** and variants thereof may be equipped with piping and pumps to transfer the content from the cartridges and/or chambers.

In some embodiments, as mentioned, the design as shown in **FIG. 5** comprises several cartridges which can be easily combined. This may, for instance, serve to increase the production volume in a particular amount of time.

**FIG. 6** illustrates another embodiment of a cartridge according to the current disclosure, showing a cross-sectional view of a cartridge, having multiple chambers 661, 662, 663, and 664 in a vertical arrangement, each provided with an inlet for reagents. In the embodiment shown in **FIG. 6****,** each chamber 661, 662, 663, and 664is at its bottom surface equipped with a valve, allowing the transfer of fluid content from a first upper chamber to a lower positioned second chamber by means of gravitation. When transfer of liquid is desired, one or more valves may receive the instruction to open to allow fluid flow. Similarly, the valves may also be instructed to close, in order to allow a (sub)-reaction in the chamber. In the embodiment shown in **FIG. 6****,** the bottom chamber 664 of the cartridge is provided with an outlet 665 for the evacuation of the content of the lower chamber. In some embodiments, the outlet 665 may be provided with one or more pipes to allow transfer of the fluid to another compartment, such as a manifold or to a downstream processing unit (not shown on **FIG. 6**). In some embodiments, the cartridge may be thus designed to allow sub-reactions taking place in one chamber, and once the sub-reaction is performed, the content of a chamber will be transferred to a lower residing chamber, allowing the further addition of reagents via the chamber inlets. Chambers that have been evacuated are then ready to obtain a subsequent sub-reaction. As such, sequential production of biochemical molecules can take place.

In some embodiments, the top chamber 661 of the cartridge can be provided by an inlet 666 for reagents on a top surface of the cartridge, or any other suitable position. It will be appreciated by a skilled person that not all chambers require the presence of inlets. In some embodiments, only the top chamber 661 can be provided by an inlet 666, whereas the bottom chamber 664 is provided with an outlet 665. In an embodiment, a feedback-loop from the outlet of the bottom chamber 664 can be provided, to one of the above positioned chambers, such as the top chamber 661.

While **FIG. 6** shows one embodiment allowing the transfer of liquid from one chamber to another, it will be appreciated that other means of fluid transfer are also possible. Such means, although not shown in the Figures, may comprise: a tubing between two successive chambers and a pumping system to move the liquid; a pressure system in each chamber and valves at the inlet and at the outlet of each chamber; a mechanical system which lifts each chamber and rotate each chamber to flow the liquid in the next chamber; a variable angular inclined plane; or any combination thereof.

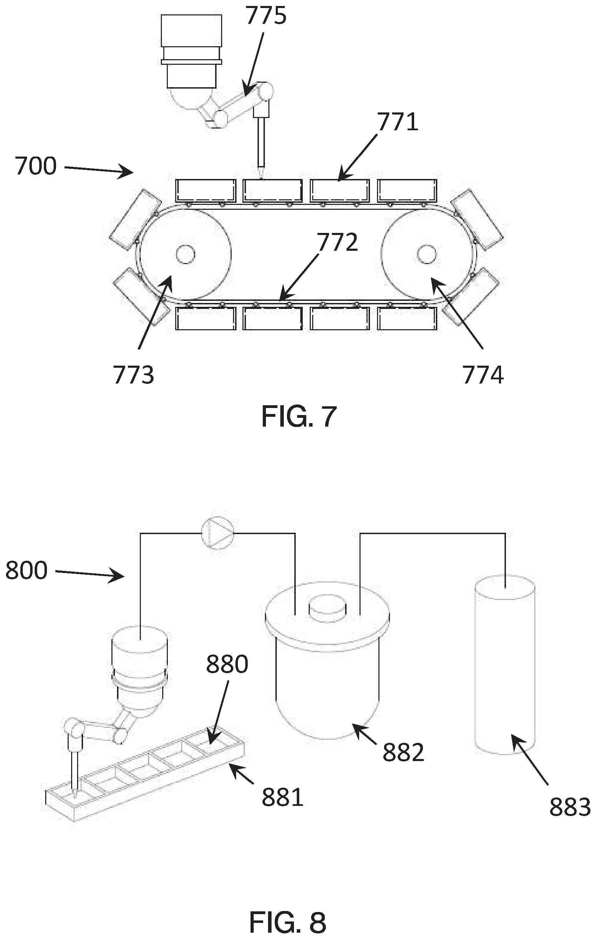

**FIG. 7** shows another embodiment of the cartridge 771, comprising the plurality of chambers (shown in cross section). In some embodiments, the chambers are configured to move on a conveyor belt 772 powered by one or more powered pulleys 773 & 774. In some embodiments, one pulley may be powered and one may be idle. In such cases, the former may be referred to as the drive pulley while the latter, unpowered pulley, may be referred to as the idler pulley.

A robotic arm 775 is provided to allow transfer of reagents to and from the chambers. A reaction may be started in a first position when the robotic arm 775 provides the necessary reagents to the chamber. The chambers are then transferred by traversing them in one or more directions until they occupy a second position. In the second position, the content of the chamber can then be removed gravitationally from the chambers. To enable reloading, the chamber is then transferred along the conveyor 772 from the second position back to the first position. The time between evacuation and reloading can be used for, e.g., disinfection and/or cleaning of the chambers.

**FIG. 8** shows a conceptual representation of the downstream processing steps that occur after one or more reactions have taken place in the cartridge 881. The result of the multiple reactions taking place in the chambers 880 is collected in a manifold. This can be done by any suitable method . The manifold 882 serves as an intermediate vessel to collect several volumes coming from the cartridge 881 and the chambers 880. Once a predefined or sufficient volume is collected in the manifold 882, the volume will then be processed in a downstream processing unit. Such downstream processing unit may comprise one or more purification devices 883 suited for purification of the product produced in the cartridges 881 and collected in the manifold 882. The downstream processing may be a batch process, such as batch purification. In another embodiment, it may also be a continuous mode process. Sufficient transfer means such as tubing, valves and pumps may be present to allow transfer of liquid to the manifold and from the manifold to the downstream processing unit.

The cartridges, manifold and downstream processing unit as described above may be embedded in a biomolecule production system. An example of such system is shown in **FIGS. 9A-****9B.** The example system is a concatenation of different modular, optionally movable, units in which different steps of the biomolecule manufacturing process are conducted. A modular unit comprises the appropriate devices and equipment for performing part or the complete biomolecule manufacturing process. In one embodiment, the equipment for upstream processing such as cartridge, robotic arm, and manifold may be provided in one unit whereas the required downstream processing devices are present in a separate unit. These units can be connected to each other.

In an embodiment, the different units may be provided with a connector, allowing the connection of the units. The connector can be configured to allow physical combination of different units by a modular connector system allowing the transfer of data. To that purpose, said connector may comprise combinations of power and signal contacts, Ethernet, optical fiber, coaxial contacts, hydraulic, pneumatic and thermocouples in a compact frame or housing. This modular connector system can be configured according to the specific requirements of the connection. The connectors can be waterproof. In an embodiment, one unit can be provided with a male connector, connectable to a female connector of a second unit. To ensure correct connection between the male and female connector, the female connector may contain centering pins. In another embodiment, the connector comprises an electronic eye to ensure correct connection. In another embodiment, the connector comprises magnetic elements to ensure correct connection.

In an embodiment, the connector is configured to couple the bioreactor cabinet to a bioreactor chamber of the production system by means of a connecting portion and receiving portion. The connecting portion may be located at the bioreactor cabinet whereas the receiving portion may be present in the bioreactor chamber of the system or vice versa.

In a further or in another embodiment, a connecting portion on a first unit and a receiving portion on a second unit will allow docking of both units to ensure that both entities are firmly connected to each other, prohibiting the release of a first unit from a second or further unit during the production of biomolecules. This connecting and receiving portion can be any suitable connecting system, such as of mechanical or magnetic system. A break-away function can be incorporated to be able to release the units.

In an embodiment, a unit may be comprised of both a connector allowing the transmission of power, signals, and/or data when paired with another unit and a magnetic connection that allows docking of a first unit to a second or subsequent unit. In an embodiment, said magnetic connection may be a permanent magnet. In another embodiment, said magnet may be an electromagnet, wherein a magnetic field is produced by an electric current. The electromagnet may allow the magnetic field to be quickly changed by controlling the amount of electric current. In some embodiments, the use of a magnet, such as an electro-magnet, enhances the safety of the system, as it may prevent unauthorized docking or removal of units. A second or subsequent unit may be comprised of a corresponding magnetic part to allow interaction with the magnet of a first unit.

The system may be provided with separate units for the delivery of reagents and the evacuation of waste, as well as for the collection of the biomolecule harvest. Each unit is thus designed to allow the provision of filtered, sterile air to be circulated within the units. Air filtering means may include for instance a HVAC system with HEPA filters.

The use of different modular units allows for the system to be assembled or constructed on site rapidly, and potentially disassembled with similar rapidity. As it is possible to easily add additional modular units, the number of cartridges, manifolds or purification units may be adjusted to suit a particular need or process setting depending on the application.

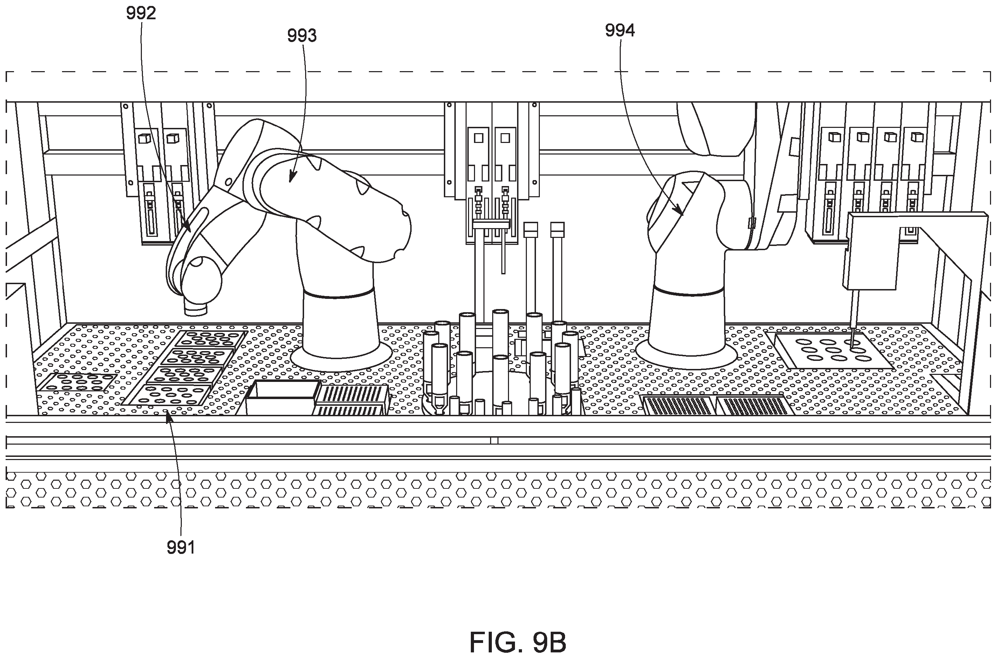

In the embodiment shown in **FIGS. 9A** and **9B****,** a central unit 990is equipped with a docking station 991 for receiving a cartridge 992 and optionally a robotic arm 993. The robotic arm 993 and the operating system may alternatively be present in the central unit 900. The central unit 900 is further provided with the appropriate equipment or docking places 994 for downstream processing of the harvest from the cartridge (e.g., chambers, cartridges, and/or robotic arms). As shown in **FIG. 9A****,** the central unit may be coupled to accessory units 995 (e.g., fridges), for instance for the provision of the necessary reagents, the collection of the end product, storage of reagents or intermediates, and/or the evacuation of waste.

One or more units may be mobile and, in such cases, provided with transportation means that allow the mobility or transportation of said bioreactor cabinet For example, in the system depicted in **FIG. 9A****,** each accessory unit 995 is provided with wheels 996. Handles 997may also be present, for easy handling of the accessory unit(s). The handle(s) make(s) it possible for an operator to move the bioreactor cabinet by pushing or pulling on the handle(s). The handle(s) can have various sizes and can be placed at different positions on the outside of a unit or portion thereof.

The housing of the units may be made of any suitable material, such as metal alloy, metal, or plastic. In one embodiment, a unit is made from a material comprising aluminum or stainless steel. In another embodiment, the bioreactor cabinet is made of a material comprising stainless steel.

The units can be designed and operated such that they only require limited handling of the operator. This is to avoid contamination and disturbance of the process conditions. If irregularities are observed, the operator can manipulate the process via one or more control devices present inside or outside the unit(s). These control devices control (e.g., parts of) the process taking place in the unit. Each unit may be coupled to one or more control devices that are configured to perform multivariate analysis, automatically control operation of the processes, and optionally, communicate with components remotely (using, for example, network protocols) in order to control operation in the unit(s).

In some embodiments, a chamber 1101 used in the methods and systems described herein comprises one or more features as shown in **FIG. 11****.** In some embodiments, the chamber 1101 comprises six flat, polygonal (e.g., rectangular) surfaces 1104. In some embodiments, the chamber comprises a cross section, the cross section (e.g. transversal section) comprising a hexagonal shape. In some embodiments, the chamber 1101 comprises a lid 1102, the lid 1102 comprising an opening 1103. In some embodiments, the lid 1102 comprises a push-on lid. In some embodiments, the opening 1103 is configured to allow for access to the chamber 1101. In some embodiments, the opening 1103 is configured to allow for access, to provide contents to the chamber 1101. In some embodiments, as shown in **FIG. 11****,** the lid comprises a hexagonal shape. In some embodiments, the chamber 1101 does not comprise a lid.

In some embodiments, as shown in **FIG. 11****,** the bottom 1105 of the chamber is pointed. In some embodiments, the bottom 1105 may be flat. In some embodiments, the bottom 1105 may be rounded (e.g., concavely or convexly).

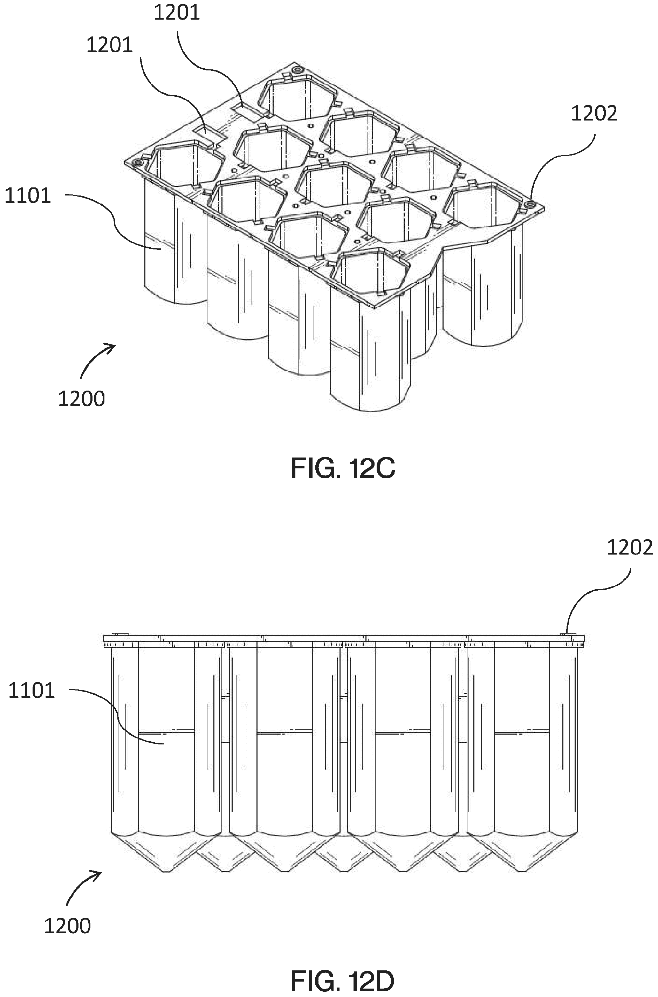

In some embodiments, chambers 1101 are comprised in one or more cartridges 1200, such as those depicted in **FIGS. 12A-12J****.** In some embodiments, the cartridges may comprise a plurality of chambers 1101 as shown in **FIGS. 12A-12J****.** In a non-limiting example shown in **FIG. 12A****,** the cartridge 1200 comprises 4 rows of chambers 1101. In another non-limiting example depicted in **FIG. 12C****,** the cartridge 1200 comprises 3 rows of chambers 1102. In some embodiments (e.g., as depicted in **FIGS. 12A-12J**), outer rows of chambers 1101 of the cartridge 1200 comprise the highest number of chambers 1101. In some embodiments, the middle rows of chambers 1101 contain fewer chambers 1101 than the outer rows of chambers 1101. The cartridge 1200 shown in the non-limiting example of **FIG. 12B** comprises chambers comprising a larger volume than the chambers shown in the non-limiting example of **FIG. 12A****.** However, the volume of a chamber 1101 may comprise any suitable value, such as those disclosed elsewhere herein. In some embodiments, the chamber comprises a volume of about 50 mL. In some embodiments, the chamber comprises a volume of about 20 mL. In some embodiments, the chamber comprises a volume of about 10 mL. In some embodiments, the chamber comprises a volume of about 5 mL. In some embodiments, the chamber comprises a volume of about 4 mL. In some embodiments, the chamber comprises a volume of about 3 mL. In some embodiments, the chamber comprises a volume of about 2 mL. In some embodiments, the chamber comprises a volume of about 1 mL.

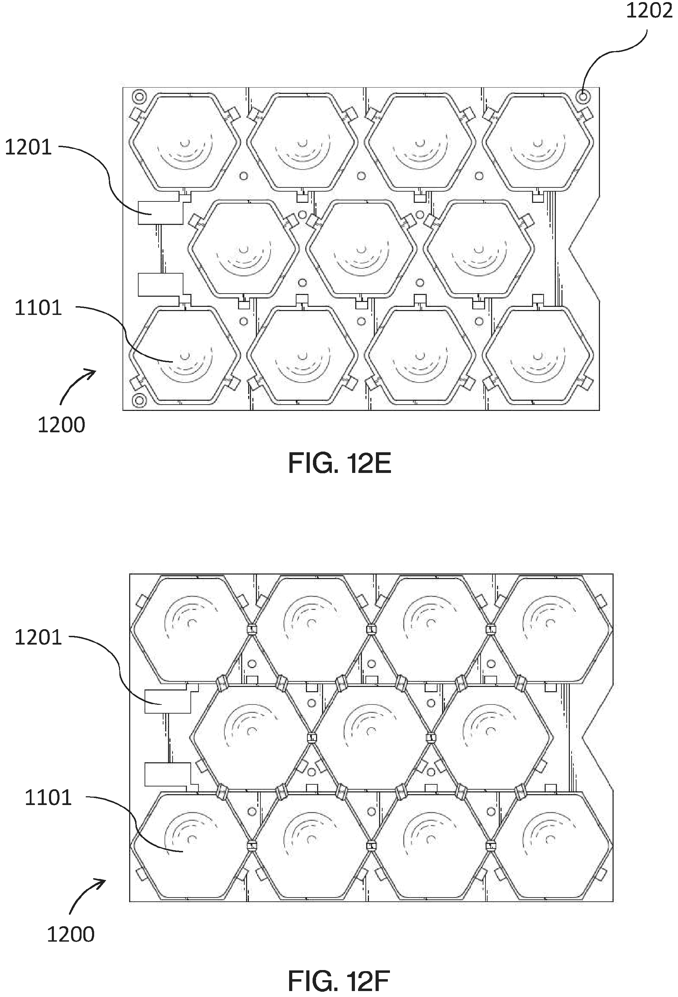

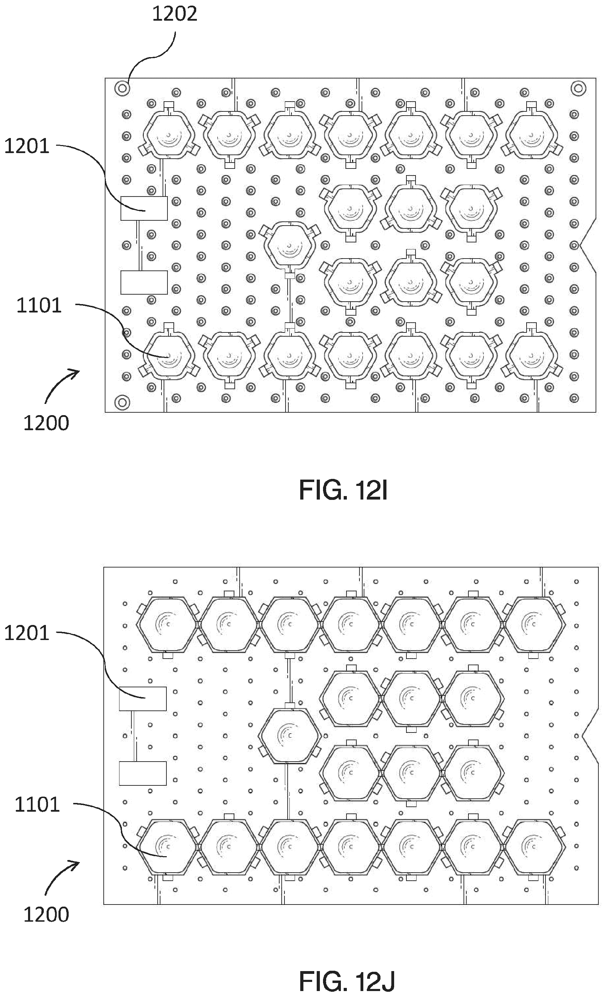

In some embodiments, the cartridge 1200 comprises an aperture 1201 as shown in the non-limiting examples of **FIG. 12A** and **12B****.** In some embodiments, the cartridges 1200 comprise at least one aperture. In some embodiments, the cartridge 1200 comprises at least two apertures 1201. In some embodiments, the cartridge is configured to engage with a gripping mechanism of a robotic arm or handling device. In some embodiments, (such as those depicted in **FIGS. 12C, 12D****,** **12E****,** and **12I**), the cartridge may comprise one or more markers 1202 to aid in positioning of the cartridge, such as within a modular unit (e.g., carousel, conveyor, heater, shaker, or combination thereof) as described above.

In some embodiments, such as those depicted in, e.g., **FIGS. 12F** and **12J****,** at least a subset of the chambers share an edge or vertex with another chamber. In some embodiments, such as those depicted in, e.g., **FIGS. 12A-12E****,** none of the chambers shares an edge or vertex with another chamber.

In some embodiments (such as those depicted in **FIGS. 12A-12B**), the chambers comprise rounded bottoms. In some embodiments (such as those depicted in **FIGS. 12C-12J**), the chambers comprise pointed bottoms.

The embodiments and devices as described above may be used in the manufacturing of biologicals. In one embodiment, devices as described herein above are used in the production of RNA by means of an in vitro transcription (IVT) reaction. Alternatively, or in a further embodiment, the embodiments may also be used for the cell-free production of protein starting from RNA (or a DNA strain that is to be transcribed). In some embodiments, the production of a biological comprises a cell-based production of protein, using, e.g., an inoculum of transiently transfected cells.

An IVT reaction as described herein may generally comprise one or more operations of: mixing a DNA template with nucleotides, DNA polymerase and other reagents; and incubating the mixture at a defined temperature for a defined duration during which: the DNA polymerase "reads" the DNA template and catalyzes the synthesis of the corresponding RNA molecule; the RNA molecule is provided with a capping structure at its 5' end by either: co-translational capping (whereby specific reagents are introduced into the reaction mix at the start of the reaction); or post-transcriptional capping (= transformation, by enzymatic action, of the starting nucleotide in the already formed RNA molecule to a capping structure comprising N⁷-methylguanosine linked to the starting nucleotide by a triphosphate). In some embodiments, an IVT reaction comprises a subset of these operations. In some embodiments, an IVT reaction comprises additional operations (e.g., downstream purification and/or isolation).

Finally, and depending on the design of the process, the transcription reaction might be followed by enzymatic digestion of the DNA template, facilitating downstream removal of DNA.

The current disclosure and its embodiments allow for the sequential production of small volumes in the chambers of a cartridge, which may subsequently be combined and processed downstream. In an embodiment, a chamber of a cartridge can be provided with reagents for allowing the production of biomolecules. A second chamber can, within a time-interval after the first chamber has been provided with reagents, also be provided with reagents. Eventually, a chain of reactions will be happening in each of the chambers, each reaction having a different starting and endpoint. Consequently, a virtual 'endless' process of production can be achieved, wherein between specific time-intervals the output of a chamber can be harvested and combined with the output of other chambers.

Finally, after production and downstream purification, the bulk drug substance is formulated and filled in individual containers (with or without additional processing steps such as lyophilization) to form the drug product.

### Examples

### Example 1: Assessing impact of rerunning reaction in the same chamber

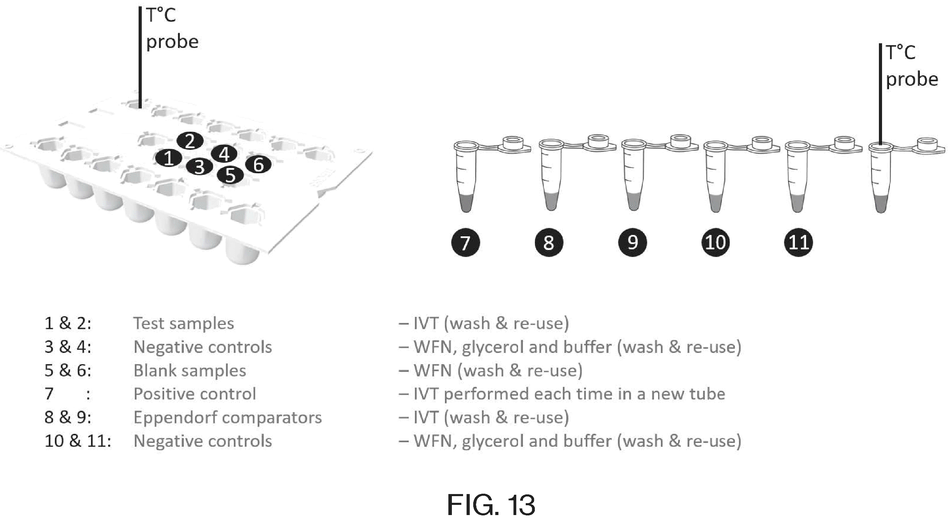

An experiment was performed in a container as described herein comprising 21 chambers, 6 of which were used for the experiment. To evaluate the reusability of the chambers, the IVT reactions were performed 12 times with washing (2 × 1 mL water) in between each reaction. An overview of the reaction set-up is depicted in **FIG. 13****.** Two chambers (samples 1 and 2 of **FIG. 13**) contained reagents and enzymes for IVT (DNA, enzymes, NTPs, CleanCap^{®} reagents, and buffer). Two chambers (samples 3 and 4 of **FIG. 13**) were negative controls with reaction mixtures comprising nuclease-free water (WFN), glycerol, and buffer-but no DNA, enzymes, NTPs, and CleanCap^{®} reagents. Two chambers were blanks comprising only WFN. In parallel, the same IVT reaction was performed in a series of Eppendorf tubes as controls. A positive control (IVT reaction, sample 7 in **FIG. 13**) was performed in a new tube each cycle. Another set of controls (samples 8 and 9 in **FIG. 13**) comprised the IVT reaction performed in the same tube each time with washing as described above in between each cycle. Finally, two tubes (reactions 10 and 11 in **FIG. 13**) comprised negative controls corresponding to samples 3 and 4, except performed in Eppendorf tubes (including washing and reuse between each IVT cycle).

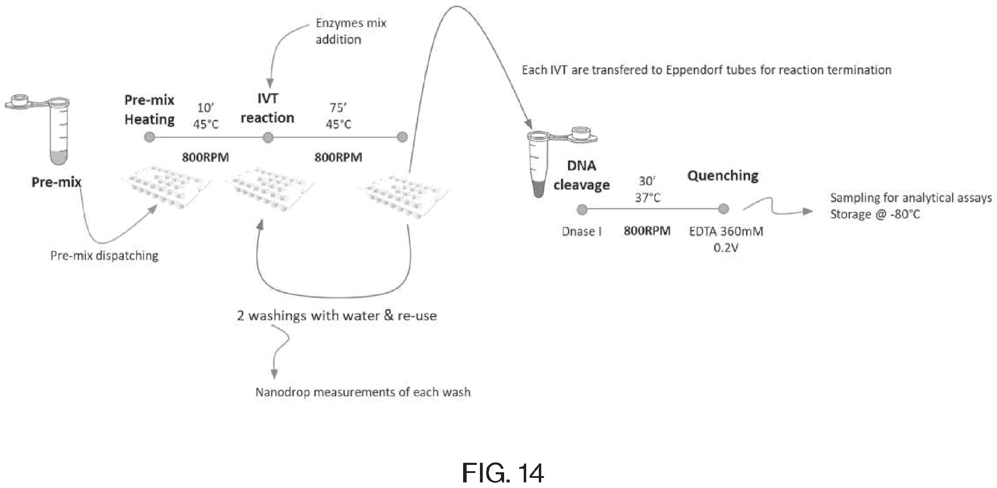

An overview of the IVT reaction is shown in **FIG. 14****.** The pre-mix was prepared and dispatched in the corresponding cartridges and Eppendorf tubes. The pre-mix was preheated for 10 min. at 45 °C before the addition of T7 RNA Polymerase (T7 Pol) mix to start IVT. IVT was allowed to proceed for 75 min with constant agitation and the reaction mixtures open to the atmosphere. Following 75 min of IVT, the contents of each IVT well/tube was transferred to a new Eppendorf tube for DNase treatment and quenching. Each IVT well/tube was then washed two times with 1 mL of water. Each wash was retained for UV-Vis spectrophotometry to evaluate possible adsorption of compounds from the reaction mixture (e.g., nucleotides, nucleic acids, proteins) to the reactor or tube wall due to repeated use.

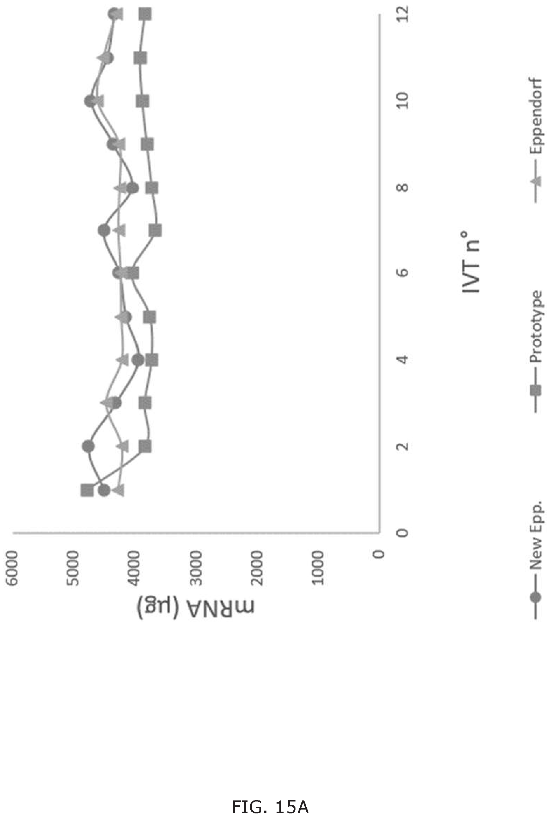

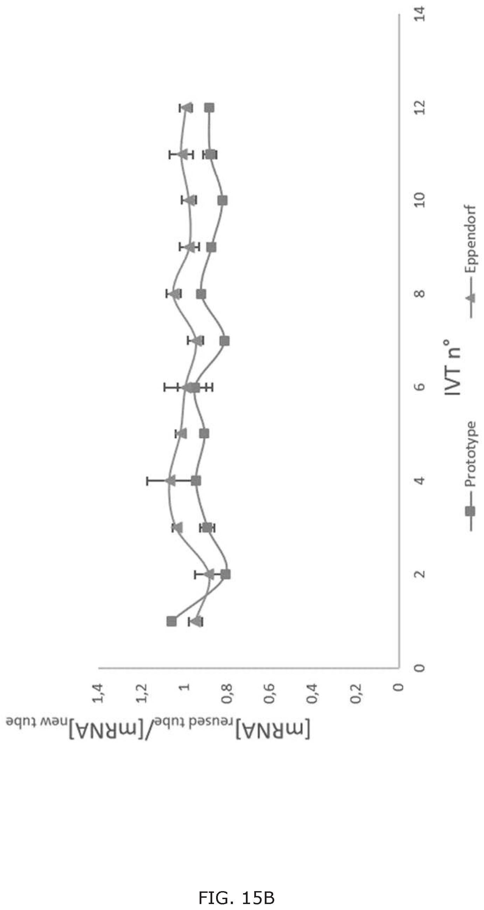

To determine changes in mRNA production as a function of cycle, the amount of mRNA produced by each reaction in each cycle was measured using a Qubit fluorescence assay. The results of the measurements are shown in **FIG. 15A****.** The ratio of mRNA produced in the chamber or reused Eppendorf tube to the corresponding new Eppendorf tube was also calculated for each reaction at each cycle and is shown in **FIG. 15B****.** As shown by **FIGS. 15A-15B****,** the amount of mRNA produced did not appear to vary as a function of the number of IVT reactions that had been performed in a given chamber.

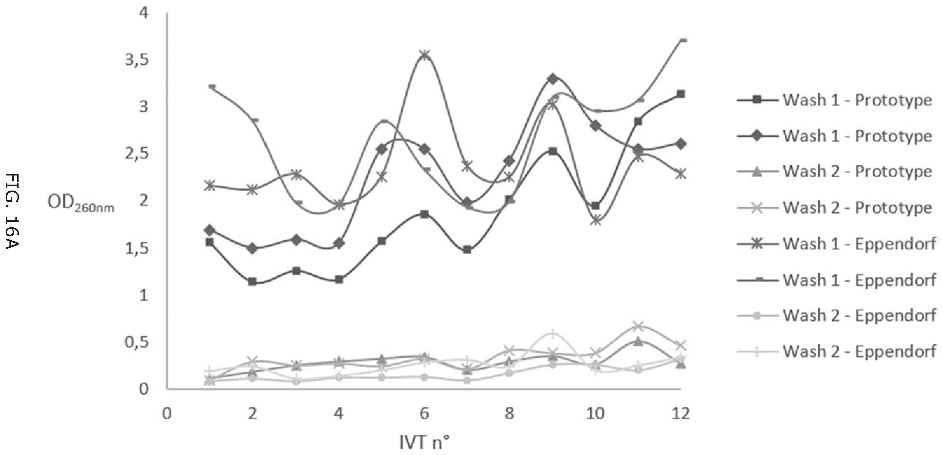

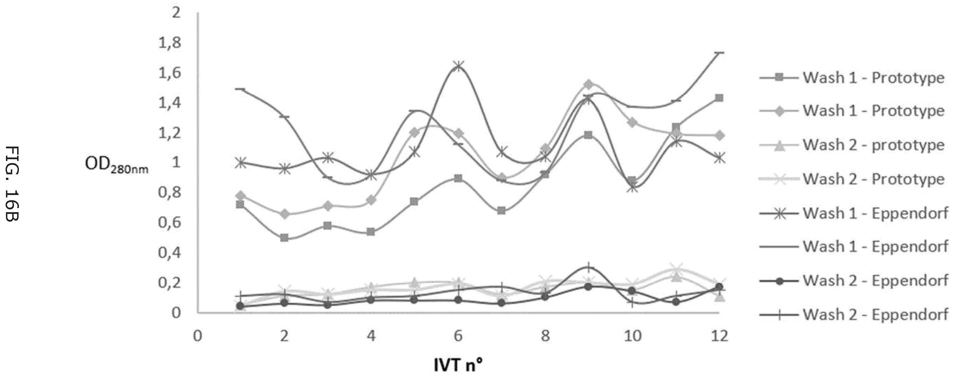

Additionally, the two washes of each cartridge were assayed by UV-Vis spectrophotometry (NanoDrop) to determine how much residual nucleic acid and/or protein was removed by washing. As shown in **FIGS. 16A-16B****,** two washes were sufficient in each case to remove residual nucleic acids (260 nm, FIG. 16A) and proteins (280 nm, FIG. 16B).

Integrity of the mRNA produced was measured as a function of reaction cycle by subjecting the synthesized RNA to fragment analysis by capillary gel electrophoresis after reactions 1, 6, and 12. The data are shown in **Table 1.** The area and relative concentration of the main peak was found to be about constant over each measurement, suggesting that the target molecule was made in each case and that reuse of the cartridges did not impact integrity of the synthesized RNA.

**Table 1. Fragment analysis of synthesized RNA**

| | IVT | Npeaks tot. | Npeak >1% | HighestPea kPerc | HighestPea kSize | 2nd | 2nd size | 3rd | 3rd size | 4th | 4th Size | % Purity | Aver age | %CV |
|---|---|---|---|---|---|---|---|---|---|---|---|---|---|---|
| Prototype R1 | 1 | 6 | 4 | 92 | 1331 | 4 | 2521 | 2 | 860 | 1 | 987 | 91.5 | 91.6 | 0.2 % |
| Prototype R2 | | 7 | 4 | 92 | 1318 | 4 | 2494 | 2 | 852 | 1 | 979 | 91.7 | | |
| Eppendorf R1 | | 7 | 4 | 92 | 1331 | 4 | 2548 | 2 | 856 | 1 | 987 | 92.1 | 92.4 | 0.5 % |
| Eppendorf R2 | | 6 | 4 | 93 | 1325 | 4 | 2521 | 2 | 852 | 1 | 979 | 92.7 | | |
| Eppendorf new | | 6 | 4 | 92 | 1325 | 4 | 2507 | 2 | 852 | 1 | 987 | 92.4 | 92.4 | N/A |
| Prototype R1 | 6 | 7 | 4 | 93 | 1325 | 4 | 2507 | 2 | 856 | 1 | 983 | 92.6 | 92.8 | 0.3 % |
| Prototype R2 | | 5 | 4 | 93 | 1331 | 4 | 2521 | 2 | 856 | 1 | 987 | 93.0 | | |
| Eppendorf R1 | | 7 | 4 | 92 | 1331 | 3 | 2507 | 2 | 856 | 1 | 983 | 92.4 | 92.8 | 0.5 % |
| Eppendorf R2 | | 6 | 4 | 93 | 1338 | 3 | 2535 | 2 | 860 | 1 | 992 | 93.1 | | |
| Eppendorf new | | 6 | 4 | 93 | 1325 | 4 | 2507 | 2 | 852 | 1 | 983 | 92.5 | 92.5 | N/A |
| Prototype R1 | 12 | 7 | 4 | 92 | 1331 | 4 | 2521 | 2 | 856 | 1 | 983 | 91.5 | 91.9 | 0.5 % |
| Prototype R2 | | 6 | 4 | 92 | 1336 | 3 | 2529 | 2 | 864 | 1 | 987 | 92.2 | | |
| Eppendorf R1 | | 5 | 4 | 93 | 1322 | 3 | 2515 | 2 | 859 | 1 | 983 | 93.2 | 93.3 | 0.1 % |
| Eppendorf R2 | | 6 | 4 | 93 | 1322 | 3 | 2529 | 2 | 855 | 1 | 983 | 93.3 | | |
| Eppendorf new | | 6 | 4 | 93 | 1329 | 2 | 2500 | 2 | 859 | 1 | 987 | 93.4 | 93.4 | N/A |

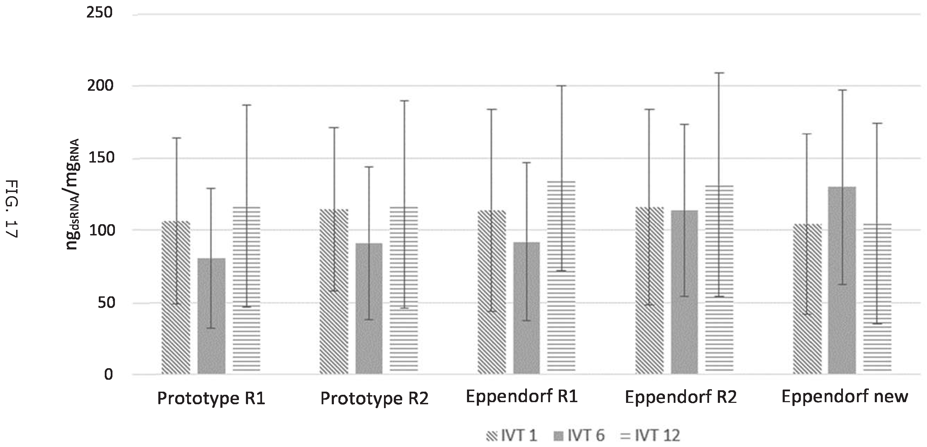

Finally, the relative proportion of double stranded RNA (dsRNA) in each sample was calculated after the 1^{st}, 6^{th}, and 12^{th} IVT reactions by and enzyme-linked immunosorbent assay (ELISA). The results are shown in **FIG. 17****.** The amount of dsRNA in each sample was seen to stay below the upper tolerance limit of 300-500 ng of dsRNA per mg of total RNA.

### Example 2. Computer Simulation of Reactor System

To model kinetic properties of a reactor system as described herein and demonstrate a continuous cycle of compound production, a computer simulation of the reactor system was performed in Microsoft Excel. The parameters of the simulation are as follows: 40 chambers of 20 mL working volume each, giving a total working volume of 800 mL; time interval between starting reactions (e.g., by adding a reagent) in subsequent chambers of 4.5 min. (time interval X = 4.5 min); time interval between start of reaction (e.g., addition of reagent) and harvesting of chamber contents (e.g., synthesized compound) of 180 min (time interval Y = 180 min); time interval between emptying contents of a chamber and adding new working volume to start a new reaction in the same chamber of 3.25 min. The number of chambers corresponds to the time interval Y divided by the time interval X (180/4.5 = 40). In practice, the addition and removal of the working volume from each chamber may be performed by devices and methods described herein above, such as one or more robotic arms configured to add and/or remove working volume from the chambers or a subset thereof.

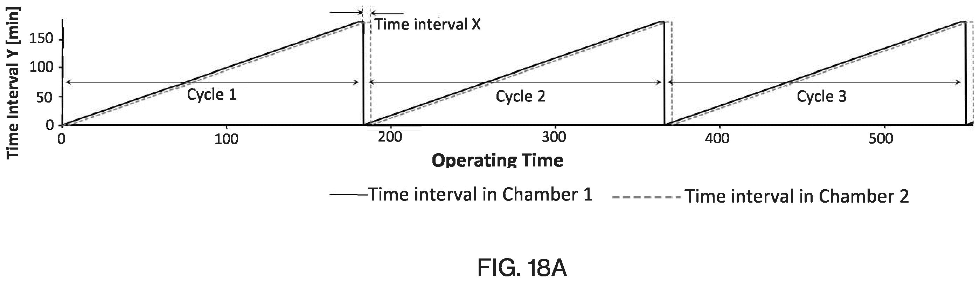

A graph of two reaction cycles in two subsequent chambers is shown in **FIG. 18A****.** The first cycle in the first chamber began at time = 0 and lasted for 180 min (time interval Y). The first cycle in the second chamber began at time = 4.5 (time interval X) and also lasted for 180 min. The first cycle in the first chamber ended at 180 min, at which point the contents of the first chamber were harvested and new contents were introduced to begin the second cycle in the first chamber. The time delay between the end of the first cycle and the start of the second cycle in the first chamber was 3.25 min, such that the second cycle in the first chamber began at time = 183.25 min. Analogously, the first cycle in the second chamber ended at time = 184.5 min, and the second cycle in the second chamber began at time = 187.25 min. As such, at all times shown in **FIG. 18A** the gap between the two curves is 4.5 min, corresponding to the time interval X. Although only three cycles (time period of at least 3Y) are depicted, this process may continue for as many cycles desired/possible (e.g., considering the need to halt production to periodically maintain the system).

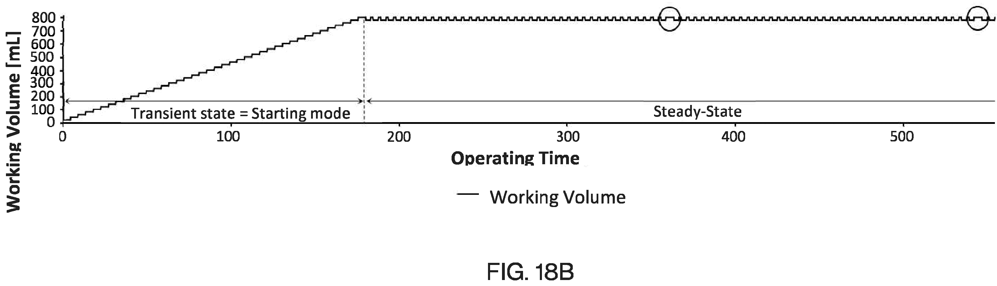

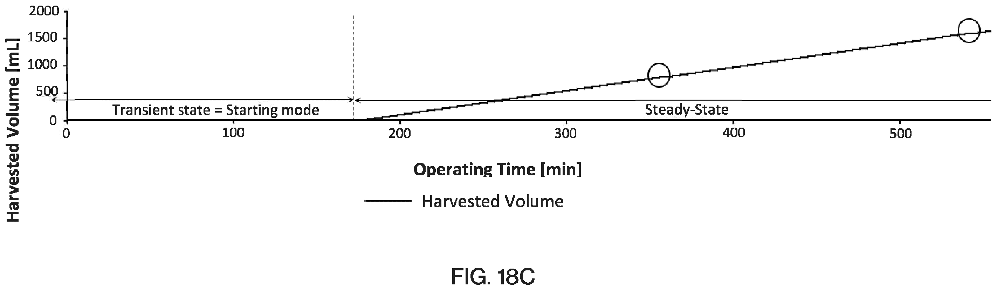

Turning from individual chambers to the bulk system level, **FIGS. 18B** and **18C** depict the total working volume in the system at a certain time and the cumulative volume harvested up to a certain time, respectively. As may be seen in **FIG. 18B****,** once a time interval Y had passed, all chambers in the system were in use, allowing the total working volume to remain about constant at about 800 mL (with some fluctuations on the order of the chamber volume (about 20 mL) due to (i) the time interval between starting reactions in subsequent chambers (time interval X) and (ii) the delay between harvesting the reaction mixture following one cycle and adding the new reaction mixture for the next cycle). Further, as depicted in **FIG. 18C****,** once the total working volume (800 mL) was in full use (after one cycle at time = 180), there was essentially continuous harvesting of material from the chambers. The reactor system, following a transient "priming" period corresponding to time interval Y, thus entered into a steady-state "production," or "running," period during which the compound was synthesized essentially endlessly at an about linear rate, with no break in operation of the system.

The foregoing has been presented for purposes of illustration and description. It is not intended to be exhaustive or to limit the embodiments to the precise form disclosed. Obvious modifications and variations are possible in light of the above teachings. All such modifications and variations are within the scope of the appended claims when interpreted in accordance with the breadth to which they are fairly, legally and equitably entitled.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

## Claims

1. A cartridge comprising one or more reaction chambers for producing a nucleic acid, each reaction chamber comprising
a bottom portion,
a body portion comprising an inner volume configured to hold at least one reagent,
and an open end configured to receive the at least one reagent;
wherein a transverse cross-section of the body portion comprises a polygonal shape.

2. The cartridge according to claim 1, wherein the transverse cross-section of the body portion of the reaction chamber is hexagonal.

3. The cartridge according to any of the claims 1 or 2, wherein the body portion of the reaction chamber comprises at least three flat polygonal surfaces.

4. The cartridge according to any of the claims 1 to 3, wherein the bottom portion of the reaction chamber is flat, pointed or rounded.

5. The cartridge according to any of the claims 1 to 4, wherein the inner volume of the reaction chamber is between 0.1 mL and 100 mL.

6. The cartridge, according to any of the previous claims, wherein the cartridge comprises a plurality of reaction chambers.

7. A cartridge comprising:
(a) a support plate; and
(b) a plurality of reaction chambers carried by the support plate, each reaction chamber having:
(i) an open end at the support plate; and
(ii) an elongated body portion extending away from the support plate,
wherein the elongated body portion has a polygonal cross-section, preferably a hexagonal cross-section, over at least a majority of its length.

8. The cartridge of claim 7, wherein each elongated body portion of the reaction chamber has a length that is at least 2 times, preferably at least 3 times, a maximum width of the elongated body portion across the polygonal cross-section.

9. The cartridge according to any of the claims 7 or 8, wherein the polygonal cross-section of the elongated body is hexagonal and the cartridge comprises at least 2 rows of reaction chambers.

10. The cartridge according to any of the claims 7 to 9, wherein the plurality of reaction chambers is arranged in a packed array in which adjacent reaction chambers are positioned such that the polygonal cross-sections tessellate without gaps, preferably in a honeycomb arrangement.

11. The cartridge according to any of the claims 7 to 10, wherein each reaction chamber has a bottom portion that is tapered and/or wedge-shaped.

12. The cartridge according to any of the claims 7 to 11, wherein the support plate comprises, for each reaction chamber, an aperture defining the open end.

13. Use of a cartridge according to any of the claims 1 to 12 for performing an in vitro transcription (IVT) reaction for producing RNA.

14. A method for producing a nucleic acid by means of a cartridge according to claims 1 to 12, comprising the steps of:
(a) providing the cartridge;
(b) introducing at least one reagent for producing the nucleic acid into a reaction chamber of the cartridge; and
(c) producing the nucleic acid in the reaction chamber;

15. The method according to claim 14, wherein step (b) further comprises shaking or oscillating the cartridge, thereby mixing the at least one reagent.
